# EUROPEAN PATENT APPLICATION

(11) **EP 2 374 459 A1**
(43) Date of publication of application: **12.10.2011**
(21) Application number: 10172550.5
(22) Date of filing: 27.05.2009
(51) Int. Cl.: A61K 31/4965, A61P 25/00

(54) **Treatment for neurological and mental disorders**

(30) Priority: 30.05.2008 US 57713 P
(62) Divisional of application: 09759052.5
(71) Applicant: Psychogenics Inc., New York 10591 (US)
(72) Inventor: The designation of the inventor has not yet been filed
(74) Representative: Andrews, Timothy Stephen

(57) **Abstract**

Methods for treating neurological or mental disorders in humans and the symptoms associated therewith are provided by administering eltoprazine and/or related compounds. In some embodiments, specific symptoms are treated by administering eltoprazine and/or a related compound in an effective amount to ameliorate the symptoms. Of particular significance are symptoms that are associated with cognitive dysfunction where eltoprazine will improve the symptoms and the disorder associated with that symptom. Of particular interest are non-ADHD-associated inattention, hyperactivity and impulsiveness. The methods provided herein are especially useful for improving functional recovery after CNS injury such as stroke where improved cognitive function will facilitate the acquisition of learning and memory of rehabilitative tasks.

## Description

The invention is directed to a novel method of treating neurological or mental disorders associated with cognitive dysfunction. The invention is also directed to methods of improving specific symptoms in an individual characterized as having a neurological or mental disorder that is associated with cognitive dysfunction.

### BACKGROUND OF THE INVENTION

Mental disorders are complex phenomena that frequently affect an individual's performance in cognitive tasks. Cognitive processes play a key role in stress-related neuropsychiatric disorders, including emotional disorders such as anxiety and depression. (Hariri et al., 2006, Trends in Cognitive Sciences., 10(4):182-191; Miles et al. 2004, Journal of Adolescence, 27(6):691-701; Waikar et al. 1997, Journal of Anxiety Disorders., 11(1):1-16). Abundant clinical and animal evidence strongly support this notion, suggesting that disturbed cognitive processes are an important part of affective illnesses. In addition, mild cognitive impairment (MCI) is significant because several disorders often present as MCI and then develop into dementia. MCI represents a transitional state between the cognitive changes associated with age-related cognitive decline and represents in most cases the earliest clinical manifestations of dementia. (Petersen, 2006 J Geriatr Psychiatry Neurol. Sep;19(3):147-54), and some vascular risk factors help enhance the risk of conversion into dementia. (Ravaglia, 2006 Dement Geriatr Cogn Disord.;21(1):51-8). Potentially treatable psychiatric disorders are also common in patients with degenerative brain diseases affecting movement and coordination. For example, one study reported that up to 80 per cent of patients with either Huntington's disease or other degenerative diseases also suffer from depression, impaired cognitive processing (e.g., thinking) and changes in personality. (Leroi et al. Am J Psychiatry. 2002 Aug;159(8):1306-14).

Parkinson's disease is a neurodegenerative disorder caused by cell death in the substantia nigra (midbrain) and which is characterized by symptoms of bradykinesia, rigidity, dyskinesia, and postural instability. Other symptoms include dementia, sleep disturbances and confusion that is associated with cognitive disorganization. Levodopa and Amantadine are two drugs used to treat some symptoms of Parkinson's disease. These drugs are directed more to the movement disorder associated with Parkinson's disease rather than the associated cognitive impairment which remains an unmet medical need.

Alzheimer's disease is a progressive neurodegenerative disorder of the CNS associated with irreversible cognitive and memory loss characterized by extracellular deposition of the amyloid-beta peptide in senile plaques, the appearance of intracellular neurofibrillary tangles, cholinergic deficits, extensive neuronal loss and synaptic changes in the cerebral cortex, hippocampus and other areas of brain essential for cognitive and memory functions. Clinical hallmarks of Alzheimer's disease are progressive impairment in attention, short-term and long-term memory, judgment, decision-making, orientation to physical surroundings, and language. It is the most common of all neurodegenerative diseases, accounting for about two-thirds of dementia cases with vascular causes, with other neurodegenerative diseases covering most of the remaining one-third. There is presently no cure for Alzheimer disease, and only four drugs are approved for the treatment of symptoms of Alzheimer's disease. There is a need for drugs with greater efficacy with fewer side effects.

Huntington's disease (HD) is a neurological disorder caused by a genetic mutation in the IT15 (huntingtin) gene. Progressive cell death in the striatum and cortex and accompanying declines in cognitive, motor, and psychiatric functions, are characteristic of the disease. This degeneration causes uncontrolled movements, loss of intellectual faculties, and emotional disturbance. Some early symptoms of HD are mood swings, depression, irritability and also trouble driving, learning new things, remembering a fact, or making a decision. Suicide is an associated risk, with suicide rates of up to 7.3 percent; four times that of the general population. Physicians prescribe a number of medications to help control emotional and movement problems associated with HD, however most drugs used to treat the symptoms of HD have side effects such as fatigue, restlessness, or hyperexcitability. In addition, these drugs are not directed at the associated cognitive impairments.

Cushing's syndrome is a disorder caused by high levels of cortisol in the blood from a variety of causes, including a pituitary adenoma (known as Cushing's disease), adrenal hyperplasia or neoplasia and ectopic adrenocorticotropic hormone (ACTH) production. Patients frequently suffer various psychological disturbances, ranging from euphoria to psychosis. Depression and anxiety are also common. Patients suffer deficits in several areas of cognition, particularly those involving processing of selective attention and visual/verbal, visuospatial learning and memory.

Lewy body disease is thought to be the second most common kind of dementia. It causes cognitive problems similar to those seen in Alzheimer's disease and motor problems like those in Parkinson's. Similar to Alzheimer's disease, Lewy body disease is currently incurable and it worsens over time. Effective treatments are lacking.

Multiple sclerosis (MS) is a debilitating neurological disease characterized by a progressive loss of motor and sensory function, which eventually leads to paralysis and death. The primary cause of neurological impairment is demyelination of nerves in the central nervous system (CNS) caused by an inflammatory autoimmune response. Thus, in people affected by MS, patches of damage called plaques or lesions appear in seemingly random areas of the CNS "white matter", which is made up of nerve fibers that are responsible for transmitting communication signals both internally within the CNS and between the CNS and the nerves supplying the rest of the body. At the site of a lesion, the nerve insulating material myelin is lost. Present therapies for MS are predominantly directed at reducing the inflammatory response responsible for the demyelination. Cognitive deficits affect the domains of memory, attention and reasoning.

Stroke (also called ischemic stroke, stroke syndrome and cerebrovascular accident) is a condition with sudden onset caused by acute vascular lesions of the brain such as infarction from hemorrhage, embolism, or thrombosis, or a rupturing aneurysm. As such, it is a heterogeneous disorder that leads to a broad range of neurological deficits for which effective therapies are urgently needed. Typical symptoms reflecting the focus of infarction or hemorrhage include hemiparesis, vertigo, numbness, aphasia and dysarthria. Most patients that survive the critical acute (hours) and sub-acute (days) phase of stroke are referred to specialized centers where they receive rehabilitative therapies. Stroke patients often display some modest degree of spontaneous improvement/recovery of lost neurological function over time. Nevertheless, this compensatory response is small, leaving significant sensory/motor impairments that lead to a persistent disability and handicap, significantly interfering with the patient's quality of life. The spontaneous functional recovery observed after cerebral infarction, particularly in the cerebral cortex, has been attributed to activation of various mechanisms of repair (i.e. axonal sprouting, angiogenesis) as well as anatomical reorganization of existing neural networks adjacent to and remote from the injured site (i.e. plasticity) (Nudo 2006; Nudo 2007).Permanent neurologic damage generally is a result, associated with various cognitive deficits such as problems with short term memory, attention, visual and verbal memory, problems speaking and understanding (dysphasia), reading and writing, all of which slow functional recovery.

Addictive Disorders are characterized by the chronic use of an agent or participation in an activity which may result in the development of tolerance, physical or psychological dependence, and finally behavioral changes to seek out the agent or activity. Addictive agents and activities include smoking, drug abuse, cocaine dependence, gambling and other impulse control disturbances. More treatment options are needed to treat symptoms and facilitate the withdrawal from the addictive agent or behavior. Psychological factors such as impaired judgment and impulsivity are at the core of these disorders.

Pervasive Developmental Disorders (PDD) refers to a group of five disorders characterized by delays in the development of multiple basic functions including socialization and communication. The most commonly known PDD is autism, and the others are Rett syndrome, childhood disintegrative disorder, Asperger syndrome, and pervasive developmental disorder not otherwise specified (or PDD-NOS). Symptoms include difficulty using and understanding language, difficulty relating to people, objects, and events, unusual play with toys and other objects, difficulty with changes in routine or familiar surroundings and repetitive body movements or behavior patterns. As such, these symptoms represent a delayed cognitive development. There is no known cure for PDD and medications are used to address certain behavioral problems.

Specific PDDs include Autism Spectrum Disorders (ASD) which are brain development disorders that impair social interaction and communication, and cause restricted and repetitive behavior, all starting before a child is three years old. Symptoms include lack of social or emotional reciprocity, stereotyped and repetitive use of language or idiosyncratic language, and persistent preoccupation with parts of objects. A person with ASD may respond atypically to medications such as antidepressants, stimulants, and antipsychotics, however the medications can have adverse effects and no known medication relieves autism's core symptoms of social and communication impairments. Cognitive disorders are severe, and another hallmark of these disorders, with a fundamental incapacity to grasp meaning, organize thoughts, and plan actions, draw relationships and attending to different stimuli being consistent symptoms.

Fragile X Syndrome is a genetic disorder caused by mutations in the FMR1 gene on the X chromosome. Symptoms include stereotypic movements (e.g., hand-flapping) and atypical social development, particularly shyness and limited eye contact. While there is no current cure for the syndrome, there is hope that further understanding of its underlying causes would lead to new therapies.

Anxiety disorders characterized by feelings of apprehension and fear, which are accompanied by physical symptoms that are severe and disabling and deficits in executive cognitive function. Symptoms of anxiety include increased respiration, tachycardia, sweating and tremor. Generally, benzodiazepines are effective in treating anxiety disorders; however, long-term use of these compounds may be limited because of associated risks for dependency.

Prader-Willi Syndrome (PWS) is a rare genetic disorder that is characterized by hyperphagia and food preoccupations, as well as small stature and learning difficulties. Symptoms include speech delay, failure to thrive, delayed milestones/intellectual delay, and hyperphagia. Prader-Willi syndrome has no cure. However, several treatments are in place to lessen the condition's symptoms. Growth hormone replacement therapy improves body composition and increases linear height.

Schizophrenia is a common and highly disabling psychiatric disorder with a population prevalence around 1%. The manifestations of schizophrenia fall into three major domains: 1) "positive" symptoms, such as delusions, hallucinations, and disorganization of behavior; 2) "negative symptoms," including social withdrawal, lack of motivation, and reduced expression of affect; and 3) cognitive dysfunction. The cognitive deficits (i.e., cognitive symptoms) include severe impairments in attention, episodic and working memory, speed of information processing and executive functioning. In a clinical trial reported by Tiihonen (Lancet 1993 Jan 30;341(8840):307) eltoprazine was administered to a group of schizophrenics for the limited purpose of assessing whether eltoprazine had any efficacy for treating aggression associated with schizophrenia.

Bipolar disorder is a category of mood disorders defined by the presence of one or more episodes of abnormally elevated mood, clinically referred to as mania. Individuals who experience manic episodes also commonly experience depressive episodes or symptoms, or mixed episodes in which features of both mania and depression are present. During manic episodes a patient may experience poor impulsive control, hyper-activity, racing thoughts, feelings of superiority and invincibility. Cognitive impairment involves poor executive function and poor episodic, emotional and verbal memory. Lithium is one of the main drugs prescribed for bipolar disorder but has a narrow therapeutic index with serious toxic side effects.

Depressive disorders affect over fifteen percent (15%) of the population. Depression is a mental state of depressed mood characterized by feelings of sadness, despair, and discouragement. Depression includes the normal feelings of "the blues" through dysthymic disorder to major depressive disorder. Dysthymic disorder is a mood disorder characterized by depressed feeling (sad, blue, low), loss of interest or pleasure in usual activities, and at least some of the following: changes in appetite and sleep patterns, lack of energy, low self esteem, poor concentration or decision-making skills, and feelings of hopelessness. In dysthymic disorders, symptoms have persisted for more than two years but are not severe enough to meet the criteria for major depressive disorder. Major depressive disorder is characterized by major depressive episodes, a period of daily depressed mood or loss of interest or pleasure in almost all activities with some combination of the following symptoms: altered appetite, weight, or sleep patterns, psychomotor agitation or retardation, diminished capacity for thinking, concentration, or decisiveness, lack of energy and fatigue, feelings of worthlessness, self-reproach, or guilt, frequent thoughts of death or suicide, plans or attempts to commit the latter (Diagnostic and Statistical Manual of Mental Disorders, 4th ed., American Psychiatric Association, Washington D.C., 1994).

Vascular Dementia refers to a group of syndromes caused by different mechanisms all resulting in vascular lesions in the brain. The most common of these is multi-infarct dementia accounting for 10-20% of all cases of progressive, or gradually worsening, dementia. Symptoms include problems with memory, difficulty with organization and solving complex problems, slowed thinking, distraction or "absent mindedness", and difficulty retrieving words from memory, difficulty following instructions, confusion, and emotional lability. Behavioral and affective symptoms are particularly important in this patient group and deserve special consideration. If these problems develop, they tend to be resistant to conventional psychopharmacological treatment and in many cases lead to hospital admission and placement in permanent care. Agents that may be useful include antidepressants, neuroleptics and mood-stabilizers.

Mild cognitive impairment (MCI) is a condition in which a person has deficits in memory, language, or another mental function severe enough to be noticeable to other people and to show up on tests, but not serious enough to interfere with daily life. There is currently no treatment for MCI approved by the FDA.

Dementia describes a progressive decline in cognitive function due to damage or disease in the brain beyond what might be expected from normal aging. In dementia, affected areas in cognition may be memory, attention, language, and problem solving. Higher mental functions are affected first in the process and in the later stages of the condition, affected persons may be disoriented in time, in place, and in person. The dementia may be classified as either conical or subcortical depending on what part of the brain is affected. Examples of dementia include, HIV-related dementia that is characterized by cognitive impairment with mental slowness, trouble with memory and poor concentration and Pick's disease which is characterized by a decreased ability to produce language both spoken and written aphasia, decreased planning capacity, mood swings, personality changes such as apathy and impulsive behavior and poor judgment. Improved medications are necessary for treating patients with this disease.

Delirium is an acute and relatively sudden decline in attention-focus, perception, and cognition. It commonly occurs in patients with dementia. Delirium is often multi-factorial and treatment is achieved by treating the underlying dysfunction causes.

There are other cognitive impairments associated with conditions for which few treatments exist, such as menopause-related memory and cognitive ability dysfunction, or cognitive deficits due to exposure to toxic or addictive compounds during early development.

United States Patent 5,424,313 relating to various piperazine compounds, including eltoprazine, states that the compounds disclosed in the 5,424,313 patent are suitable for the treatment of affections or diseases which are the result of disturbances in the central nervous system, for example, psychoses, aggression, fear, depression, etc. Some of the compounds are stated as having central analgesic activity. Development of eltoprazine as a treatment for aggression in humans was stopped following unsatisfactory results from human clinical trials. (See Verhoeven et al., 1992, The Lancet, 340:1037-1038; Tiihonen, 1993, The Lancet, 341:307; Kohen, 1993, The Lancet, 341:628-629; Moriarty et al., 1994, Human Psychopharm., 9:253-258; DeKoning et al., 1994, Int. Clin. Psychopharm, 9:187-194; Oliver, 1994, Prog. Drug Res. 42:167-308). More recently, eltoprazine has been found to be useful for treating ADHD and ADHD-associated symptoms. (See U.S. patent publication 2003/0050308 incorporated by reference in its entirety herein.)

The binding profile of eltoprazine, together with the direct binding data obtained with [³H] eltoprazine, shows the compound to be a selective 5-HT₁ ligand (selective with respect to all receptors other than 5-HT₁). Eltoprazine's binding affinity for the various 5-HT receptor subtypes closely resembles serotonin except for the relatively low affinity for the 5-HT_{1D} receptor with roughly equipotent affinity for the 5-HT_{1A}, 5-HT_{1B}, and somewhat lower affinity for 5-HT_{2C} receptors (Schipper, J. et al., supra). Eltoprazine acts as a mixed 5-HT_{1A/1B} receptor agonist. Eltoprazine has no relevant affinity for dopamine receptors (i.e., Kᵢ>1 µM, Schipper et al., supra). Among the 5-HT receptors, the 5-HT_{1B} receptor is located as an autoreceptor on axon terminals and is responsible for inhibiting neurotransmitter release, whereas it is also located postsynaptically as a heteroreceptor on axons and terminals of non-serotonergic neurons inhibiting their activity.

What is therefore needed are novel pharmaceutical compounds for treating cognitive impairment associated with various neurological and mental disorders as well as symptoms in an individual characterized as having these disorders. Also needed are novel formulations and methods of treating neurological and mental disorders, as well as symptoms in an individual characterized as having these disorders.

### SUMMARY OF INVENTION

This invention relates to the use of eltoprazine hydrochloride and/or related compounds to treat cognitive impairment associated with neurological or mental disorders and symptoms thereof, in particular, one or more of hyperactivity, inattention, and/or impulsivity, as well as conditions that manifest themselves in other disorders. There are a number of neurological and mental disorders that result in impairment of cognitive tasks and treatment with eltoprazine and/or related compounds may improve symptoms of these disorders and the disorders themselves. These neurological or mental disorders include, but are not limited to, Parkinson's disease, Alzheimer's disease, Huntington's disease, Cushing's disease, Lewy body disease, multiple sclerosis, stroke, addictive disorders (for example smoking, drug abuse, cocaine dependence, gambling and other impulse control effects), pervasive, development disorder, autism, fragile X syndrome, anxiety disorders (e.g. acute and chronic panic, post traumatic stress disorder, generalized anxiety disorder), Prader-Willi syndrome, schizophrenia unassociated with aggression, bipolar disorder, depression, vascular dementia, mild cognitive impairment, dementia, amnestic disorders, delirium and other cognitive impairments.

The invention disclosed herein is particularly efficacious for preventing and/or treating specific symptoms associated with the neurologic component of certain disorders. Specifically, in one embodiment, eltoprazine is particularly useful for improving cognitive function in individuals having impairments due to any of inattention or hyperactivity or impulsivity, or combinations thereof.

Methods and compositions useful for treating neurological and mental disorders associated with cognitive dysfunction characterized by inattention, and/or hyperactivity and/or impulsivity, but not ADHD, include, for example:

i) Parkinson's disease

ii) Alzheimer's disease

iii) Huntington's disease

iv) Cushing's disease

v) Lewy Body disease

vi) multiple sclerosis

vii) stroke

viii) addictive disorders (for example smoking, drug abuse, cocaine dependence, gambling and other impulse control effects)

ix) pervasive developmental disorder

x) autism

xi) Fragile X syndrome

xii) anxiety disorders (e.g. acute and chronic panic, post traumatic stress disorder, generalized anxiety disorder)

xiii) Prader-Willi syndrome

xiv) non-aggressive schizophrenia

xv) bipolar disorder

xvi) depression

xvii) vascular dementia

xiii) mild cognitive impairment

xix) dementia

xx) delirium

xxi) other conditions associated with cognitive impairment

It is understood that treatment of each of the foregoing represents a distinct embodiment of the invention. The invention also provides treatment of the foregoing conditions, or underlying symptoms of each as well as inattention, hyperactivity or impulsiveness associated with these conditions. The present invention further provides methods for treating cognitive symptoms of the neurological or mental disorders, which include the administration of eltoprazine and/or a related compound. The compounds for use in the invention are believed to be effective in the treatment of neurological or mental disorders and exhibit reduced side effects and are not expected to have abuse potential, as compared to other available therapeutics.

Treatment of neurological or mental disorders according to this invention may be used to reduce one or more of any of the diagnostic criteria associated with these conditions. In a preferred embodiment of this invention, eltoprazine is administered to individuals characterized as having a neurological or mental disorder, other than ADHD, to provide treatment of symptoms associated with impaired cognitive function. In one embodiment, such symptoms are inattention and/or hyperactivity and/or impulsivity. Treatment is often evaluated using clinical evaluation methods and tests that are known in the art to determine improvement of such cognitive symptoms. One object of the invention is to provide a method for treating neurological or mental disorders by administering to an individual a therapeutically effective amount of a compound of the following formula: 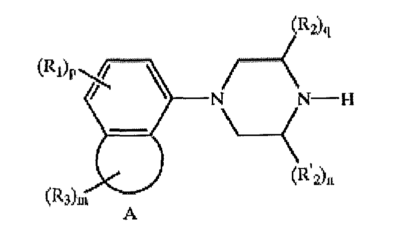 wherein
R₁ is hydrogen, alkyl, cycloalkyl, optionally esterified hydroxyalkyl, alkoxyalkyl, optionally substituted phenyl or heteroaryl, alkoxy, aryloxy, alkylthio, arylthio, acyl, alkoxycarbonyl, aminocarbonyl, alkyl- or dialkyl-aminocarbonyl, nitro, amino, alkyl- or dialkyl-amino, acylamino, alkylsulfonylamino, arylamino, cyano, halogen, trifluoromethyl, trifluoromethoxy, optionally esterified hydroxyl, alkyl- or amino-sulphonyl or -sulphinyl, alkyl- or dialkyl-aminosulphonyl or -sulphinyl, and p has the value 0-3;
-R₂ and R'₂ are independently hydrogen or an alkyl group and n and q can have the value 0 or 1;
-R₃ may have the same meaning as R₁, or is alkylidene, an oxo or thioxogroup, and m has the value 0-2;
-A forms, with the two carbon atoms of the phenyl group, an optionally entirely or partly unsaturated cyclic group having 5-7 atoms in the ring, which comprises 1-3 hetero atoms from the group O, S, and N, with the proviso that the sum of the number of oxygen and sulphur atoms is at most 2; and
wherein
- the compound may be a racemate or a single diastereomer or enantiomer;
- a pharmaceutically acceptable acid addition salt thereof.

Another object of the invention is to provide a method for treating neurological or mental disorders by administering to an individual a therapeutically effective amount of a compound of the following formula: 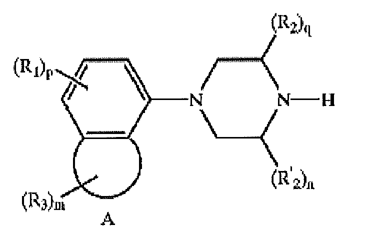 wherein
R₁ is hydrogen, alkyl, cycloalkyl, optionally esterified hydroxyalkyl, alkoxyalkyl, optionally substituted phenyl or heteroaryl, alkoxy, aryloxy, alkylthio, arylthio, acyl, alkoxycarbonyl, aminocarbonyl, alkyl- or dialkyl-aminocarbonyl, nitro, amino, alkyl- or dialkyl-amino, acylamino, alkylsulfonylamino, arylamino, cyano, halogen, trifluoromethyl, trifluoromethoxy, optionally esterified hydroxyl, alkyl- or amino-sulphonyl or -sulphinyl, alkyl- or dialkyl-aminosulphonyl or -sulphinyl, and p has the value 0-3;
-R₂ and R'₂ are independently hydrogen or an alkyl group and n and q can have the value 0 or 1;
-R₃ may have the same meaning as R₁, or is alkylidene, an oxo or thioxogroup, and m has the value 0-2;
-A forms, with the two carbon atoms of the phenyl group, an optionally entirely or partly unsaturated cyclic group having 6 atoms in the ring, which comprises 1-3 hetero atoms from the group O, S, and N, with the proviso that the sum of the number of oxygen and sulphur atoms is at most 2; and
wherein
- the compound may be a racemate or a single diastereomer or enantiomer;
- a pharmaceutically acceptable acid addition salt thereof.

It is therefore an object of the invention to provide methods and compositions for treating neurological and mental disorders associated with cognitive impairment individuals without ADHD. In addition, the present invention provides a method for improving cognitive function in such individuals characterized as having neurological and mental disorders other than ADHD.

### BRIEF DESCRIPTION OF THE DRAWINGS

FIG. 1: Eltoprazine (0.1mg/kg IP) sharpens the peak of responding and significantly reduces the spread. There was no loss of effect following chronic administration.

FIG. 2: Administration of 0.5 mg/kg IP scopolamine resulted in decreased entries to repeat in the radial arm maze compared to baseline, and this effect was attenuated by administration of 0.3 mg/kg PO eltoprazine, as revealed by ANOVA.

FIG. 3: Eltoprazine at 0.3, 1, 3, and 10 mg/kg administered 1 h prior to training significantly improved rat novel object recognition (NOR) memory as compared to vehicle-treated controls 24 hours post training. The positive control galantamine, at 3 mg/kg, also significantly increased NOR memory relative to saline.

FIG. 4: Six mice per dose group were treated with vehicle or eltoprazine at 3 mg/kg, 10 mg/kg and 30 mg/kg. Eltoprazine exhibited a dose-related increase in the release of dopamine in the prefrontal cortex (PFC) of C57 mice.

FIG. 5: Six mice per dose group were treated with vehicle or eltoprazine at 3 mg/kg, 10 mg/kg and 30 mg/kg. Eltoprazine exhibited a dose-related increase in the release the dopamine metabolite, DOPAC in the PFC of C57 mice.

FIG. 6: Six mice per dose group were treated with vehicle or eltoprazine at 3 mg/kg, 10 mg/kg and 30 mg/kg. Eltoprazine exhibited a dose-related increase in the release of the norepinephrine metabolite, HVA in the PFC of C57 mice.

FIG. 7: Six mice per dose group were treated with vehicle or eltoprazine at 3 mg/kg, 10 mg/kg and 30 mg/kg. Eltoprazine exhibited a dose-related increase in the release of norepinephrine in the PFC of C57 mice.

FIG. 8: Six mice per dose group were treated with vehicle or eltoprazine at 3 mg/kg, 10 mg/kg and 30 mg/kg. Eltoprazine exhibited a dose-related increase in the release of serotonin in the PFC of C57 mice.

FIG. 9: Six mice per dose group were treated with vehicle or eltoprazine at 3 mg/kg, 10 mg/kg and 30 mg/kg. Eltoprazine exhibited a dose-related increase in the release of the serotonin metabolite, 5HIAA in the PFC of C57 mice.

### DETAILED DESCRIPTION OF THE INVENTION

This invention provides a method of treating cognitive impairment, other than ADHD, in individuals, by administering to individuals a therapeutically effective amount of the compound according to the formula below: 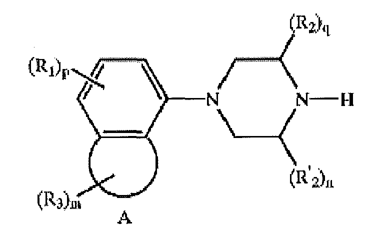 wherein
R₁ is hydrogen, alkyl, cycloalkyl, optionally esterified hydroxyalkyl, alkoxyalkyl, optionally substituted phenyl or heteroaryl, alkoxy, aryloxy, alkylthio, arylthio, acyl, alkoxycarbonyl, aminocarbonyl, alkyl- or dialkyl-aminocarbonyl, nitro, amino, alkyl- or dialkyl-amino, acylamino, alkylsulfonylamino, arylamino, cyano, halogen, trifluoromethyl, trifluoromethoxy, optionally esterified hydroxyl, alkyl- or amino-sulphonyl or -sulphinyl, alkyl- or dialkyl-aminosulphonyl or -sulphinyl, and p has the value 0-3;
-R₂ and R'₂ are independently hydrogen or an alkyl group and n and q can have the value 0 or 1;
-R₃ may have the same meaning as R₁, or is alkylidene, an oxo or thioxogroup, and m has the value 0-2;
-A forms, with the two carbon atoms of the phenyl group, an optionally entirely or partly unsaturated cyclic group having 5-7 atoms in the ring, which comprises 1-3 hetero atoms from the group O, S, and N, with the proviso that the sum of the number of oxygen and sulphur atoms is at most 2; and
wherein
- the compound may be a racemate or a single diastereomer or enantiomer;
- a pharmaceutically acceptable acid addition salt thereof.

In another embodiment, this invention provides a method of treating cognitive impairment, other than ADHD, in individuals, by administering to individuals a therapeutically effective amount of a compound of the following formula: 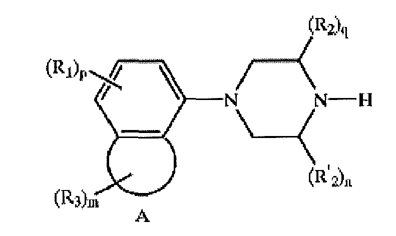 wherein
R₁ is hydrogen, alkyl, cycloalkyl, optionally esterified hydroxyalkyl, alkoxyalkyl, optionally substituted phenyl or heteroaryl, alkoxy, aryloxy, alkylthio, arylthio, acyl, alkoxycarbonyl, aminocarbonyl, alkyl- or dialkyl-aminocarbonyl, nitro, amino, alkyl- or dialkyl-amino, acylamino, alkylsulfonylamino, arylamino, cyano, halogen, trifluoromethyl, trifluoromethoxy, optionally esterified hydroxyl, alkyl- or amino-sulphonyl or -sulphinyl, alkyl- or dialkyl-aminosulphonyl or -sulphinyl, and p has the value 0-3;
-R₂ and R'₂ are independently hydrogen or an alkyl group and n and q can have the value 0 or 1;
-R₃ may have the same meaning as R₁, or is alkylidene, an oxo or thioxogroup, and m has the value 0-2;
-A forms, with the two carbon atoms of the phenyl group, an optionally entirely or partly unsaturated cyclic group having 6 atoms in the ring, which comprises 1-3 hetero atoms from the group O, S, and N, with the proviso that the sum of the number of oxygen and sulphur atoms is at most 2; and
wherein
- the compound may be a racemate or a single diastereomer or enantiomer;
- a pharmaceutically acceptable acid addition salt thereof.

In a preferred embodiment, the compound is eltoprazine. Eltoprazine's utility for treating the disorders described herein and symptoms associated therewith, is based on the discovery disclosed herein that eltoprazine selectively reduces certain symptoms associated with ADHD to a greater degree than others. Specifically, in human clinical studies, eltoprazine had greater activity treating hyperactivity and inattention compared to impulsivity in patients with ADHD. This selective response suggests that eltoprazine and/or related compounds having similar pharmacologic profiles, are useful for treating other cognitive disorders having similar symptoms.

In one embodiment, this invention provides methods for treating cognitive impairment associated with inattentiveness and/or hyperactivity and/or impulsivity. As used herein, the term impaired cognitive function refers to impairments of thinking, reasoning and judgment as determined by evaluation methods known to one of ordinary skill in the art. "Cognitive impairment" includes, but is not limited to, an acquired deficit in one or more of memory function, problem solving, orientation and abstraction. "Cognitive function testing" may fall into the categories of attention related tasks such as simple reaction time, choice reaction time, and digit vigilance; categories of working memory such as numeric working memory and spatial working memory; categories of secondary episodic recognition memory testing, such as word recognition, picture recognition, immediate word recall, and delayed word recall; as well as other tasks such as visual tracking. Other examples of standard tests for measuring cognitive impairment may include, but are not limited to, the Mini Mental State Examination, the Global Deterioration Scale and Geriatric Depression Scale, the Randt Memory Test, and the Alzheimer's Disease Assessment Scale. The term "disorder", unless stated otherwise, has the same meaning as the terms "condition" and "disease" and are used interchangeably throughout the description and claims.

In a further embodiment, the invention provides methods for the treatment or attenuation of a symptom of a neurological or mental disorder associated with cognitive dysfunction or impairment, other than ADHD. In some embodiments, the invention provides methods for the treatment or attenuation of a symptom of a neurological or mental disorder without co-morbid ADHD. In some such non-limiting embodiments, the disease or disorder is Parkinson's Disease, Alzheimer's Disease, Huntington's Disease, Cushing's Disease, Lewy body disease, multiple sclerosis, stroke, addictive disorders (for example smoking, drug abuse, cocaine dependence, gambling and other impulse control effects), autism, anxiety disorders (e.g. acute and chronic panic, post traumatic stress disorder, generalized anxiety disorder), schizophrenia, bipolar disorder, depression, vascular dementia, mild cognitive impairment, dementia, delirium, amnestic disorders, and other cognitive impairments including cognitive disorder not otherwise specified (e.g., mild neurocognitive disorder and postconcussional disorder), although other neurological or mental disorders associated with cognitive dysfunction or impairment are also contemplated for the methods described herein.

In one embodiment, the methods disclosed herein are used to treat Parkinson's disease. Parkinson's disease is a neurodegenerative disorder caused by cell death in the dopaminergic substantia nigra. In some embodiments, the present invention provides methods for treating one or more symptoms of Parkinson's disease. Examples of such symptoms include but are not limited to speech changes, loss of facial expression, cognitive dysfunction, mood swings, emotional lability, euphoria, bipolar syndrome, anxiety, aphasia, dysphasia, or disturbances, dementia or confusion, depression, fear, anxiety, memory difficulties, slowed thinking, sexual dysfunction, fatigue, aching, and loss of energy.

In one embodiment, the methods disclosed herein are used to treat Alzheimer's disease. Alzheimer's disease is a progressive neurodegenerative disorder of the CNS associated with irreversible cognitive and memory loss characterized by extracellular deposition of the amyloid beta peptide in senile plaques, and extensive neuronal loss. In another embodiment, the invention provides methods for treating one or more symptoms of Alzheimer's disease. Symptoms include, but are not limited to, impairment in memory, attention, judgment, decision-making, orientation to physical surroundings, language, speed-dependent activities, abstract reasoning, visuospatial abilities, executive functioning, and behavioral disturbances, disinterest and passivity, apathy, inappropriate dressing, poor self care, agitation, violent outbursts, aggression, depression, anxiety, hallucinations, delusions, changes in personality and mood changes, and dementia. In one embodiment, treatment of cognitive symptoms attention and working memory is provided, whilst potential for decreased aggression and improved affect also exists.

In one embodiment, the methods disclosed herein are used to treat Huntington's disease (HD). Huntington's disease is a neurological disorder caused by a genetic mutation in the IT15 gene. Progressive cell death in the striatum and cortex, and accompanying declines in cognitive, motor, and psychiatric functions, are characteristic of the disease. In some embodiments, the present invention provides methods for treating one or more symptoms of Huntington's disease . Some early symptoms of HD are mood swings, depression, irritability or trouble driving, learning new things, remembering a fact, or making a decision.

In one embodiment, the methods disclosed herein are used to treat Cushing's syndrome. Cushing's Syndrome is a disorder caused by high levels of cortisol in the blood from a variety of causes, including a pituitary adenoma (known as Cushing's disease), adrenal hyperplasia or neoplasia, ectopic adrenocorticotropic hormone (ACTH) production. In some embodiments, the present invention provides methods for treating one or more symptoms of Cushing's syndrome. Symptoms include various psychological disturbances, ranging from euphoria to psychosis. Depression and anxiety are also common.

In one embodiment, the methods disclosed herein are used to treat Lewy Body Disease. Lewy body disease causes cognitive problems similar to those seen in Alzheimer's disease. Similar to Alzheimer's disease, Lewy body disease is currently incurable and it worsens over time. In another embodiment, the invention provides methods for treating one or more symptoms of Lewy Body Disease. Symptoms include dementia, cognitive problems (problems with thinking, memory, language, varying levels of alertness and attention, visual hallucinations, and delusions).

In one embodiment, the methods disclosed herein are used to treat multiple sclerosis. Multiple sclerosis is an autoimmune condition in which the immune system attacks the central nervous system, leading to demyelination of nerve fibers. It may cause numerous physical and mental symptoms, and often progresses to physical and cognitive disability. In another embodiment, the invention provides methods for treating one or more symptoms of multiple sclerosis. Symptoms include depression, cognitive dysfunction, dementia, mood swings, emotional lability, euphoria, bipolar syndrome, anxiety, aphasia, dysphasia, and fatigue.

In one embodiment, the methods disclosed herein are used to treat stroke. Stroke is the rapidly developing loss of brain functions due to a disturbance in the blood vessels supplying blood to the brain. This can be due to ischemia caused by thrombosis or embolism, or due to a hemorrhage. In another embodiment, the invention provides methods for treating one or more symptoms of stroke. Symptoms of stroke include hemiparesis, vertigo, numbness, aphasia, confusion, depression, fatigue, sensory motor deficits, dysarthria, dysphasia, facial drooping, loss of balance or coordination, inability to walk, changes in sensation and vision problems. Nontraditional symptoms include, e.g., headache, disorientation and change in consciousness. Stroke rehabilitation is the process by which patients with disabling strokes undergo treatment to help them return to normal life as much as possible by regaining and relearning the skills of everyday living. Functional recovery after stroke requires good visual motor function and coordination, requiring executive functions such as attention and working memory. In another embodiment, the invention provides methods for facilitating the functional recover after stroke by improving cognitive function.

In one embodiment, the methods disclosed herein are used to treat addictive disorders. Addictive Disorders are characterized by the chronic or habitual use of an agent or participation in a behavior which may result in the development of tolerance, physical or psychological dependence, and finally behavior changes to seek out the agent or behavior. In some embodiments, the present invention provides methods for treating one or more symptoms of addictive disorders. Symptoms of addictive disorders include physical or emotional dependency.

In one embodiment, the methods disclosed herein are used to treat pervasive developmental disorders (PDD). PDD refers to a group of five disorders characterized by delays in the development of multiple basic functions including socialization and communication. The most commonly known PDD is autism, and the others are Rett syndrome, childhood disintegrative disorder, Asperger syndrome, and pervasive developmental disorder not otherwise specified (or PDD-NOS). In some embodiments, the present invention provides methods for treating one or more symptoms of PDD. Symptoms include difficulty using and understanding language, difficulty relating to people, objects, and events, unusual play with toys and other objects, difficulty with changes in routine or familiar surroundings and repetitive body movements or behavior patterns.

In one embodiment, the methods disclosed herein are used to treat autism and autism spectrum disorders. Autism and Autism Spectrum Disorders are brain development disorders that impairs social interaction and communication, and causes restricted and repetitive behavior, all starting before a child is three years old. In some embodiments, the present invention provides methods for treating one or more symptoms of autism and autism spectrum disorders. Symptoms of autism include aggression, agitation, delayed or unusual speech patterns, high pitched or flat intonation, lack of slang, difficulty understanding tone of voice and body language as a way of expression, lack of eye contact, inability to take another's perspective, hypersensitivity or hyposensitivity to light, sound, crowds and other external stimulation and fine or gross motor difficulty.

In one embodiment, the methods disclosed herein are used to treat anxiety disorders Fragile X Syndrome. Fragile X Syndrome is a genetic disorder caused by mutation of the FMR1 gene on the X chromosome. In some embodiments, the present invention provides methods for ameliorating one or more symptoms of anxiety disorders. Symptoms include stereotypic movements (e.g., hand-flapping) and atypical social development, particularly shyness and limited eye contact. While there is no current cure for the syndrome, there is hope that further understanding of its underlying causes would lead to new therapies.

In one embodiment, the methods disclosed herein are used to treat anxiety disorders. Anxiety disorders are characterized by feelings of apprehension and fear, which are accompanied by physical symptoms that are severe and disabling. In some embodiments, the present invention provides methods for ameliorating one or more symptoms of anxiety disorders. Examples of such symptoms include, but are not limited to, feelings of apprehension and fear, which are accompanied by physical symptoms that may reflect a category of anxiety disorder. For example, symptoms of Generalized Anxiety Disorder (GAD) include, e.g., trembling, muscle aches, insomnia, abdominal upsets, dizziness and irritability. Obsessive-Compulsive Disorder (OCD) is symptomized by, e.g., persistent, recurring thoughts (obsessions), which may lead the individual to perform ritual or routine behavior (compulsions). Panic Disorder symptoms include, e.g., heart palpitations, chest pain, chest discomfort, sweating, trembling, tingling sensations, feeling of choking, fear of losing control, fear of dying, and feelings of unreality. Three main symptoms are associated with Post-Traumatic Stress Disorder (PTSD), which are (1) "reliving" the traumatic event, such as flashbacks, nightmares, intrusive thoughts and recollections, (2) avoidance behaviors and emotional numbing, and (3) hypersensitivity such as an inability to sleep, anxious feelings, overactive startle response, hypervigilance, imitability and outbursts of anger. Physical symptoms of Social Anxiety Disorder include, e.g., heart palpitations, faintness, blushing and profuse sweating.

In one embodiment, the methods disclosed herein are used to treat Prader-Willi Syndrome (PWS). PWS is a rare genetic disorder that is characterized by hyperphagia and food preoccupations, as well as small stature and learning difficulties. In another embodiment, the invention provides methods for ameliorating one or more symptoms of PWS. Symptoms include speech delay, failure to thrive, delayed milestones/intellectual delay, and hyperphagia.

In one embodiment, the methods disclosed herein are used to treat schizophrenia unrelated to aggression. Schizophrenia is a disorder characterized by three distinct symptom clusters. In another embodiment, the invention provides methods for ameliorating one or more symptoms of schizophrenia unassociated with aggression. Examples of positive symptoms of schizophrenia include, but are not limited to, hallucinations, delusions and/or paranoia. Examples of negative symptoms of schizophrenia include, but are not limited to, social withdrawal, flat affect, anhedonia and/or decreased motivation. In still further embodiments of the methods of the invention, the symptom of schizophrenia treated according to the invention is associated with a deficit in cognitive functioning. Examples of such cognitive symptoms include, but are not limited to, severe deficit in attention, object naming, working memory, long-term memory storage or executive functioning, a slowing of information processing or neural activity, or long term depression.

In one embodiment, the methods disclosed herein are used to treat bipolar disorder. Bipolar disorder is a category of mood disorders defined by the presence of one or more episodes of abnormally elevated mood, clinically referred to as mania. In some embodiments, the present invention provides methods for treating one or more symptoms of bipolar disorder related to impaired cognitive functioning. Symptoms of bipolar disorder include depression symptoms such as depressed mood, loss of interest or pleasure in some or all activities, changes in appetite, weight or sleep patterns, lack of energy, fatigue, low self esteem, diminished capacity for thinking, concentration, or decisiveness, negative neural bias, misappropriated attention, impulsivity, self harm, feelings of hopelessness or worthlessness, psychomotor agitation or psychomotor retardation, self-reproach, inappropriate guilt, frequent thoughts of death or suicide (suicidality), plans and/or attempts to commit suicide. Symptoms of bipolar disorder also include manic symptoms such as poor impulse control, hyperactivity, poor information, processing speeds, inflated self-esteem or grandiosity, decreased need for sleep, more talkative than usual, flight of ideas, attention is easily drawn to unimportant items, increase in goal-directed activity or psychomotor agitation and excessive involvement in pleasurable activities that have a high potential for painful consequences.

In one embodiment, the methods disclosed herein are used to treat depression. Depression is a mental state of depressed mood characterized by feelings of sadness, despair, and discouragement. In another embodiment, the invention provides methods for treating one or more symptoms of depression associated with cognitive functioning. Symptoms include depressed feeling or mood, loss of interest or pleasure in some or all activities, changes in appetite, weight or sleep patterns, lack of energy, fatigue, low self esteem, diminished capacity for thinking, concentration, or decisiveness, negative neural bias, misappropriated attention, self harm, feelings of hopelessness or worthlessness, psychomotor agitation or psychomotor retardation, self-reproach, inappropriate guilt, frequent thoughts of death or suicide (suicidality), plans and/or attempts to commit suicide.

In one embodiment, the methods disclosed herein are used to treat vascular dementia. Vascular Dementia refers to a group of syndromes caused by different mechanisms all resulting in vascular lesions in the brain. In some embodiments, the present invention provides methods for treating one or more symptoms of vascular dementia. Symptoms of vascular dementia include problems with recent memory, confusion, emotional lability, and difficulty following instructions.

In one embodiment, the methods disclosed herein are used to treat mild cognitive impairment (MCI). MCI is considered to be the boundary or transitional stage between normal aging and dementia. In some embodiments, the present invention provides methods for treating one or more symptoms of MCI. Symptoms of MCI include deficits in memory, language, or another mental function that severe enough to be noticeable to other people and to show up on tests, but not serious enough to interfere with daily life.

In one embodiment, the methods disclosed herein are used to treat dementia. Dementia is a progressive decline in cognitive function due to damage or disease in the brain beyond what might be expected from normal aging. In some embodiments, the present invention provides methods for treating one or more symptoms of dementia. Symptoms of dementia include disorientation, problems with memory, attention, language, and problem solving.

In one embodiment, the methods disclosed herein are used to treat delirium. Delirium is an acute and relatively sudden (developing over hours to days) decline in attention-focus, perception, and cognition. In some embodiments, the present invention provides methods for treating one or more symptoms of delirium. Symptoms of delirium include memory deficit, disorientation, language disturbance.

The methods disclosed herein are also useful for treating other conditions associated with cognitive impairments not specifically described herein. Examples of other conditions include, but are not limited to, menopause-related memory and cognitive ability dysfunction, or cognitive deficits due to exposure to toxic or addictive compounds during early development.

For all the conditions described herein, one of ordinary skill in the art will appreciate how to determine the presence or absence of characteristic symptoms and also how to diagnose these conditions. A number of criteria for diagnosing disease are useful for characterizing these conditions such as for example, NINCDS-ADRDA criteria (McKhann et al., 1984), the ICD-10 criteria (World Health Organization, 1992), and/or the DSM-IV criteria (American Psychiatric Association, 1994). Other manuals useful in diagnosing the conditions described herein include for example, but are not limited to Oppenheimer's Diagnostic Neuropathology: A Practice Manual (Esiri and Perl, 2006, Hodder Arnold, London.); Harrison's Principles of Internal Medicine (Ed. Kasper et al, 16th Ed.2005 McGraw Hill, Columbus, OH); Goetz: Textbook of Clinical Neurology (Eds. Goetz, Pappert, 2nd Ed. 2003, W.B. Saunders, Philadelphia, PA). One of ordinary skill will be aware of other such manuals routinely used in the art to diagnose these conditions.

In some such embodiments, a patient is identified as having a cognitive symptom of the neurological or mental disorder, and is administered a therapeutically effective amount of eltoprazine, and/or a related compound and/or a pharmaceutically acceptable salt thereof

Unlike certain other therapeutics which have the potential to be abused and/or have undesirable side effects, the present invention is not expected to have the abuse potential of psychostimulants, the most widely prescribed current pharmacological treatment, and may have a side effect profile distinct from other types of pharmacologic therapeutics.

As discussed above, the disorders are diagnosed based on an individual possessing symptoms as defined according to the DSM-IV-TR and other methods recognized in the art. The compounds for use with this invention, preferably eltoprazine, may be used to treat the disorders described herein, and/or the specific symptoms or various combinations of the constellation of symptoms associated with them. In one embodiment, "treatment" or "treating" refers to an attenuation, prophylaxis, or reversal of a disease or disorder, or at least one discernible symptom thereof.

In another embodiment, "treatment" or "treating" refers to an attenuation, prophylaxis, or reversal of at least one measurable physical or cognitive parameter, not necessarily discernible in or by the subject. In yet another embodiment, "treatment" or "treating" refers to inhibiting or slowing the progression of a disease or disorder, either physically or cognitively, e.g., stabilization of a discernible symptom, physiologically, e.g., stabilization of a physical or cognitive parameter, or both. In yet another embodiment, "treatment" or "treating" refers to delaying the onset of a disease or disorder, for example, by inhibiting underlying pathological processes before they reach clinical significance.

In another embodiment, compounds related to eltoprazine are useful in the methods of the invention. In this embodiment, the methods of treatment of the disorders described herein according to this invention are accomplished by administering to an individual in need of treatment a therapeutically effective amount of a compound of the following formula: 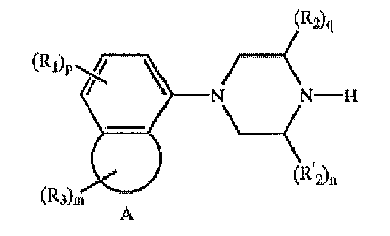 wherein
- R₁ is hydrogen, alkyl, cycloalkyl, optionally esterified hydroxyalkyl, alkoxyalkyl, optionally substituted phenyl or heteroaryl, alkoxy, aryloxy, alkylthio, arylthio, acyl, alkoxycarbonyl, aminocarbonyl, alkyl- or dialkyl-aminocarbonyl, nitro, amino, alkyl- or dialkyl-amino, acylamino, alkylsulfonylamino, arylamino, cyano, halogen, trifluoromethyl, trifluoromethoxy, optionally esterified hydroxyl, alkyl- or amino-sulphonyl or -sulphinyl, alkyl- or dialkyl-aminosulphonyl or -sulphinyl, and p has the value 0-3;

- R₂ and R'₂ are independently hydrogen or an alkyl group, and n and q can have the value 0 or 1;
- R₃ may have the same meaning as R₁, or is alkylidene, an oxo or thioxogroup, and m has the value 0-2;
- A forms, with the two carbon atoms of the phenyl group, an optionally entirely or partly unsaturated cyclic group having 5-7 atoms in the ring, which comprises 1-3 hetero atoms from the group O, S, and N, with the proviso that the sum of the number of oxygen and sulphur atoms is at most 2.

Unless otherwise defined, an alkyl is 1-10 carbons, aryl is 6-10 carbons, and cycloalkyl is 3-10 carbons.

When a halogen, R₁ is preferably fluoro, chloro or bromo, and when an alkyl group, R₁ is preferably a straight or branched, saturated or unsaturated group having 1-5 carbon atoms.

When an alkyl group, R₂ is preferably a methyl or ethyl group.

When a hydroxyalkyl group, R₃ preferably comprises 1-3 carbon atoms.

When R₁ or R₃ is an esterified hydroxyl group or hydroxylalkyl group, the ester group preferably has the formula O-CO-R₄ or -O-CS-R₄ in which R₄ is alkyl, aralkyl, aryl, heteroaryl, hetero aralkyl, wherein the alkyl group may be branched or unbranched, and the (hetero) aryl part may optionally be substituted, or R₄ may be an alkoxy, heteroalkoxy or dialkylamino group, in which the two alkyl groups can form a hetero-cyclic ring with the nitrogen atom.

When R₁ or R₃ is an etherified hydroxyl group or hydroxyalkyl group, the ether group preferably has the formula -O-R₅, wherein R₅ is a straight, branched or cyclic alkyl group having 1-5 C-atoms, or an alkoxyalkyl group having 1 or 2 C-atoms in both the alkoxy part and in the alkyl part thereof.

Eltoprazine (1-(2,3-dihydro-1, 4-benzodioxanyl-5-yl) piperazine) is particularly preferred for use with this invention: R₁, R₂, R'₂ and R₃ are hydrogen and A, together with the phenyl ring to which it is attached, forms a 2,3-dihydro-1,4-benzodioxin, C₁₂H₁₆N₂O₂; or pharmaceutically acceptable salts thereof, preferably HCl. Another preferred compound that may be useful for this invention is batoprazine, (8-(1-piperazine)-2H-1-benzopyran-2-one). This invention also includes the use of prodrugs of the compounds of the formulas provided, specifically derivatives of the compounds of the formulas that are inactive but are converted to an active form in the body following administration.

The compounds described above including eltoprazine and their methods of synthesis are known in the art and are described in U.S. Pat. No. 4,833,142; U.S. Pat. No. 5,424,313; European Patent No. 189,612; and European Patent No. 138,280, each of which is incorporated herein by reference in its entirety.

The dose of the compound used in treating the disorders described herein in accordance with this invention will vary in the usual way with the seriousness of the disorder, the weight, and metabolic health of the individual in need of treatment. The preferred initial dose for the general patient population will be determined by routine dose-ranging studies, as are conducted, for example, during clinical trials. Therapeutically effective doses for individual patients may be determined, by titrating the amount of drug given to the individual to arrive at the desired therapeutic or prophylactic effect, while minimizing side effects. A preferred initial dose for this compound may be from about 0.1 mg/day to about 20 mg/day. More preferably, the initial dose is estimated to be from about 0.1 mg/day to about 10 mg/day. Even more preferred, the initial dose is estimated to be from about 0.5 mg/day to about 5 mg/day. Even more preferred, the initial dose is estimated to be from about 1.0 mg/day to about 5 mg/day. Most preferred is from about 2.5 to about 5 mg/day, inclusive.

Other useful doses of eltoprazine and/or related compounds are from about 0.5 to about 20 mg/day, from about 1.0 to about 15 mg/day, from about 5 to about 10 mg/day, from about 7 to about 12 mg/day and from about 8 to about 10 mg/day. In some embodiments, daily doses of eltoprazine and/or related compounds are 0.1 mg, 0.5 mg, 1 mg, 2 mg, 2.5 mg, 5 mg, 7 mg, 9 mg, 10 mg, 12 mg, 15 mg, 17 mg, 19 mg, 20 mg. Administration schedules may also be altered to achieve a therapeutically effective concentration of compound to treat the disorder or symptoms described herein. In some embodiments, the compound may be administered once per day, twice per day, thrice per day, 4 times per day, 5 times per day, 7 times per day or 10 times per day. Often the dosage is divided equally throughout the day, however in some embodiments to treat certain disorders or symptoms, it may be useful to bias the dosage administration schedule so that most of the daily treatment is administered at the beginning half of the day. In some embodiments, about 50% 60% , 70% or 80% of the dosage is administered in the first half of the day. In other embodiments, it may be more appropriate to administer most of the dosage in the latter half of the day so that about 50%, 60% , 70% or 80% of the dosage is administered in the latter half of the day.

In another embodiment, the eltoprazine and/or related compound is co-administered with another therapeutic compound. The adjunctive compound may have actions that are similar to, synergistic to or different than eltoprazine and/or related compounds. Examples of adjunct compound include, but are not restricted to antipsychotic agents, mood stabilizers, SSRI antidepressants (including St. John's Wort), a 5HT_{1A} receptor agonist, or other serotonin mediated treatment, narcotic analgesics, anticoagulants, antiemetics, beta blockers, sedative antihistamines, NSAIDs (e.g., aspirin and ibuprofen); COX-2 inhibitors; synthetic and natural opiates (e.g., oxycodone, meperidine, morphine, and codeine); mexiletine; baclofen; tramadol; antiarrhythmics; anticonvulsants (e.g., lamotrigine, gabapentin, valproic acid, topiramate, famotodine, phenobarbital, diphenylhydantoin, phenytoin, mephenytoin, ethotoin, mephobarbital, primidone, carbamazepine, ethosuximide, methsuximide, phensuximide, trimethadione, benzodiazepines such as diazepam, phenacemide, acetazolamide, progabide, clonazepam, divalproex sodium, magnesium sulfate injection, metharbital, paramethadione, phenytoin sodium, valproate sodium, clobazam, sulthiame, dilantin, diphenylan); capsaicin cream; membrane-stabilizing drugs (e.g., lidocaine); N-methyl-D-aspartate receptor (NMDA) antagonists such as ketamine; as well as all other known analgesic drugs and drugs useful for treating symptoms of neuropathies, such as pregabalin, harkoseride, amitriptyline, desipramine and other related tricyclic antidepressants, and any drug with central nervous system activity.

Administration of the compounds of this invention may be by any method used for administering therapeutics, such as for example oral, parenteral, intravenous, intramuscular, subcutaneous, or rectal administration.

It will be appreciated by one of ordinary skill in the art that age of the patient with the conditions described herein may respond to treatment at different degrees depending on factors such as dosage or administration or the presence of other factors or co-morbid conditions. Therefore, one of ordinary skill in the art will appreciate that the methods described herein may be directed to a particular age group. Treatment groups may be divided by age into infants/toddlers, children, adolescents, adults and seniors and geriatrics. As used herein, these terms refer to approximately the following age groups: infants/toddlers (about age 5 and younger), children (from about age 5 to about age 12 years), adolescents (from about age 12 to about age 22 years), adults (from about age 22 to about age 55 years), seniors (from about age 55 to about age 65 years) and geriatrics (about age 65 and older). As used herein, the term infant refers generally to a human from birth until the time of assumption of erect posture. As used herein, the term toddler refers generally to a young child and the time during which the child begins to learn about social roles and develops motor skills. As used herein, the term adolescent refers generally to the period of life beginning with the appearance of secondary sex characteristics and terminating with the cessation of somatic growth. As used herein, the term adult refers generally to an individual who has attained full growth or maturity. As used herein, the term senior refers generally to an adult individual who has not yet reached the geriatric stage, but who has begun to age. As used herein, the term geriatric refers generally to an adult individual who has reached old age.

In addition to comprising the therapeutic compounds for use in this invention, especially eltoprazine [1-(2,3-dihyro-1, 4-benzodioxin-5-yl) piperazine] or pharmaceutically acceptable salts (preferably HCl in the case of eltoprazine) or pro-drug thereof, the pharmaceutical compositions for use with this invention may also comprise a pharmaceutically acceptable carrier. Such carriers may comprise additives, such as preservatives, excipients, fillers, wetting agents, binders, disintegrants, buffers may also be present in the compositions of the invention. Suitable additives may be, for example magnesium and calcium carbonates, carboxymethylcellulose, starches, sugars, gums, magnesium or calcium stearate, coloring or flavoring agents, and the like. There exists a wide variety of pharmaceutically acceptable additives for pharmaceutical dosage forms, and selection of appropriate additives is a routine matter for those skilled in art of pharmaceutical formulation.

The compositions may be in the form of tablets, capsules, powders, granules, lozenges, suppositories, reconstitutable powders, or liquid preparations such as oral or sterile parenteral solutions or suspensions.

In order to obtain consistency of administration it is preferred that a composition of the invention is in the form of a unit dose. Unit dose forms for oral administration may be tablets, capsules, and the like, and may contain conventional excipients such as binding agents, for example syrup, acacia, gelatin, sorbitol, tragacanth, or polyvinylpyrrolidone; and carriers or fillers, for example lactose, sugar, maize-starch, calcium phosphate, sorbitol or glycine. Additives may include disintegrants, for example starch, polyvinylpyrrolidone, sodium starch glycolate or microcrystalline cellulose; preservatives, and pharmaceutically acceptable wetting agents such as sodium lauryl sulphate.

In addition to unit dose forms, multi-dosage forms are also contemplated to be within the scope of the invention. Modified or controlled release dosage forms are contemplated for use in the invention, including, but not limited to sustained release dosage forms, extended release dosage forms, delayed release dosage forms, and pulsatile release dosage forms.

Suitable polymers for use in the controlled release formulations of the present invention include but are not limited to uncrosslinked, linear polymers including cellulosic polymers, preferably hydroxyethyl cellulose, sodium carboxymethyl cellulose, hydroxypropylmethyl cellulose and hydroxypropyl cellulose, microcrystalline cellulose, methyl cellulose, and ethyl cellulose, and combinations thereof; covalently crosslinked insoluble polymers such as high molecular weight crosslinked homopolymers and copolymers of (meth) acrylic acid including carbopol resins, or mixtures of these uncrosslinked and covalently crosslinked polymers. Additionally suitable polymers include acrylic acid, methacrylic acid, methyl acrylate, ammonio methylacrylate, ethyl acrylate, methyl methacrylate and/or ethyl methacrylate, vinyl polymers and copolymers such as polyvinyl pyrrolidone, polyvinyl acetate, polyvinylacetate phthalate, vinylacetate crotonic acid copolymer, and ethylene-vinyl acetate copolymers, to name a few. Various combinations of two or more of the above polymers are also contemplated for use in the dosage forms of the invention.

Delayed release compositions may be prepared, for example, by employing slow release coatings, micro encapsulation, and/or slowly dissolving polymers.

The solid oral compositions may be prepared by conventional methods of blending, filling, tabletting or the like. Repeated blending operations may be used to distribute the active agent throughout those compositions employing large quantities of fillers. Such operations are conventional in the art. The tablets may be coated according to methods well known in normal pharmaceutical practice, for example with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol syrup, methyl cellulose, gelatin, hydroxyethylcellulose, carboxymethylcellulose, aluminum stearate gel, and hydrogenated edible fats; emulsifying agents, for example lecithin, sorbitan monooleate, or acacia; nonaqueous vehicles (which may include edible oils), for example almond oil or fractionated coconut oil, oily esters such as esters of glycerine, propylene glycol, or ethyl alcohol; preservatives, for example methyl or propyl p-hydroxybenzoate or sorbic acid; and if desired conventional flavoring or coloring agents.

For parenteral administration, fluid unit dosage forms are prepared utilizing the compound and a sterile vehicle, and, depending on the concentration used, can be either suspended or dissolved in the vehicle. In preparing solutions, the compound can be dissolved in water or saline for injection and filter sterilized before filling into a suitable vial or ampoule and sealing. Advantageously, additives such as a local anesthetic, preservative and buffering agent can be dissolved in the vehicle. Suitable buffering agents are, for example, phosphate and citrate salts. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilized by conventional means, for example by exposure to radiation or ethylene oxide, before being suspended in the sterile vehicle. Advantageously, a surfactant or wetting agent is included in the composition to facilitate uniform distribution of the compound.

Patients can be evaluated for neurological or mental disorders by any of the tests known in the art. Treatment for Parkinson's disease can be assessed by measuring the reduction of neurotoxicity of MPTP in rats (Lee E H et al., 1992 Chin J Physiol 35(4):317-36). Also experimentally induced striatal DA depletion in animals is a valid model of Parkinsonism (Schultz W 1982 Prog Neurobiol 18(2-3): 121-66). The capacity of certain substances to damage catecholaminergic neurons has been used extensively to produce DA deficiency in animals (Annett L E et al., 1994 Exp Neurol 125(2): 228-46).

Cognitive symptoms of memory, including those from neurodegenerative diseases for example Alzheimer's disease, Huntington's disease and vascular disease such as stroke, can be evaluated by such models as the Five-choice Serial Reaction Time Test, (The 5-choice serial reaction time task: behavioural pharmacology and functional neurochemistry. Psychopharmacology (Berl). 2002 Oct;163(3-4):362-80). Fear Conditioning Paradigm (Gould T J et al., 2002 Behav Pharmacol. 13(4):287-94; Hamm A O et al., 2003 Brain 126 (Pt 2):267-75) and novel object recognition (Animal Models of Cognitive Impairment, 2006 (Ed. By Levin, E. D. & Buccafusco, J. J.). Boca Raton, Fla.) while spatial reference memory and learning can be evaluated in the Radial Arm Test (Aggleton J P et al., 1996 Behav Brain Res. 19(2):133-46)or Morris watermaze (Bontempi B et al., 1996 Eur J Neurosci. 8(11):2348-60). Additionally, memory and hippocampal hypo-functioning can be assessed by measuring measuring the impact of eltoprazine on synaptic plasticity (Day and Good, 2005 Jan., Neurobiol Learn Mem., 83(1): 13-21). A number of transgenic animal models may be used in combination with these paradigms and tests such as the Tg2576, APP, PS1 and PS2 transgenic mice. (Frautschy SA ,Am J Pathol. 1998 Jan;152(1):307-17).

Huntington's disease can be evaluated using behavioral and cognitive tests in combination with transgenic models such as the N171-82Q and R6/2 mouse models of Huntington's disease. (Luthi-Carter, Hum Mol Genet. 2000 May 22;9(9):1259-71; Cha, Proc Natl Acad Sci U S A. 1998 May 26;95(11):6480-5).

Cushing's disease can be evaluated by behavioral and cognitive tests in combination with such models as the spontaneous horse and dog models (Kemppainen, Trends Endocrinol Metab. 1994 Jan-Feb;S(1):21-8.).

Lewy body disease can be evaluated by behavioral and cognitive tests in combination with such models as behavioural tests in combination with transgenic models such as the alpha-synuclein transgenic mouse. (Kahle, Am J Pathol. 2001 Dec;159(6):2215-25).

Multiple sclerosis can be evaluated by the experimental autoimmune encephalomyelitis (EAE) model (Liu H Y et al., 2002 J Neurosci Res 70(2): 238-48; Lublin, Springer Semin Immunopathol. 1985;8(3):197-208).

Further for stroke, the Tamura model is one of the best-characterized focal ischemia models whereby the middle cerebral artery is occluded by electro-coagulation. Also the Johnson and McCarty model, the spontaneously hypertensive rat (SHR), and the newer endothelin-1 model may be used for evaluating stroke (Johnson M P, McCarty D R et al., 1998 Life Sci. 63(4):241-53; Sharkey J and Butcher S P 1995 J Neurosci Methods 60(1-2):125-31).

Addictive disorders can be evaluated using behavioral and cognitive tests in combination with such models as the reinstatement model (Shaham, Psychopharmacology (Berl) 168, 3-20 (2003), reinforcement learning models such as the temporal difference reinforcement learning (TDRL) model (Redish, Science. 2004 Dec 10;306(5703):1944-7).

Autism spectrum disorders can be evaluated using behavioral and cognitive tests in combination with such models as R451C transgenic mice (Tabuchi, Science. 2007 Oct 5;318(5847):71-6.).

Anxiety disorders can be evaluated using behavioral and cognitive tests in combination with such models as desert hedgehog knockout mice (Umehara, Behav Brain Res. 2006 Nov 1;174(1):167-73).

Preclinically, animals can be evaluated for blockade/attenuation of symptoms associated with schizophrenia unassociated with aggression. Positive symptoms in animal models of schizophrenia can be evaluated by measuring changes in the overall level of activity of dopamine (DA) activity with concomitant parallel changes in locomotor activity (Depoortere R et al., 2003 Neuropsychopharmacology 28(11):1889-902), D-amphetamine (AMPH) and phencyclidine (PCP) via induction of model psychosis or locomotor hyperactivity (Freed W J et al., 1984 Neuropharmacology 23(2A):175-81. For example, Depoortere et al. have described tests for evaluating locomotor activity, catalepsy, climbing and stereotypy, which relate to positive symptomatology and side effect profile, by characterizing compounds with typical and atypical antipsychotic efficacy (2003). Attenuation in apomorphine-induced climbing, stereotypy and catalepsy (AIC) can be evaluated as described by Fung Y K et al. 1986 Pharmacol Biochem Behav. 1986 24(1):139-41 and Fung, et al, 1987 Steroids 49(4-5):287-94. Additional models that can be used to assess symptoms of schizophrenia include the social isolation rearing model (Geyer et al., Biol. Psychiatry, 34, 361-372, 1993) and the maternal deprivation model (Ellenbroek et al., Schizophr. Res., 30(3), 251-260, 1998). A widely accepted means of validating animal models of schizophrenia includes the prepulse inhibition test (Van den Buuse et al., Curr. Mol. Med., 3, 459-471, 2003). Further, changes in attention function because of schizophrenia can be examined by the five (5) Choice Serial Reaction Time Test (5CSRT) (see Muir J L, et al., 1995 Psychopharmacology (Berl) 118(1): 82-92; Robbins et al., 1998 Ann N Y Acad Sci. 846:222-37), and the attentional set shifting test (Birrell, J Neurosci. 2000;20:4320-4324), working memory test (Egan, Proc Natl Acad Sci USA 2001 98: 6917-6922) and executive function (Ho, Mol Psychiatry 2005 10: 287-298). Changes in social cognition can also be examined using the social recognition test.

Bipolar disorder can be evaluated using behavioral and cognitive tests in combination with such models as D-box binding protein (Dbp) mice (Le-Niculescu H, Am J Med Genet B Neuropsychiatr Genet. 2008 Mar 5;147(2):134-66) or the amphetamine rodent model of bipolar disorder (Frey, Life Sci. 2006 Jun 13;79(3):281-6).

An assessment of depression can be measured using models such as 5-HT_{1A} knockout mice (Pattij, Behavioural Brain Research, 2003;141(2):137-145), and the chlorpyrifos (CPF) developmental exposure model (Aldridge, Environ Health Perspect. 2005 May;113(5):527-31). Depression and anxiety can both be evaluated by tail suspension-induced disuse atrophy in ovariectomized rats (Ohmori S et al., 2001 Environ Med 45(1):12-4). Further, anxiety may be assessed by the following tests: (1) the Geller-Seifter conflict test (Babbini M et al., 1982 Pharmacol Biochem Behav 17(1): 43-8; Shimizu H et al., 1992 Jpn J Pharmacol 58(3): 283-9), (2) social interaction (Gonzalez L E et al., 1998 Pharmacol Biochem Behav 59(4): 787-92), (3) light/dark exploration (Holmes A et al., 2001 Behav Brain Res 122(2): 159-67), (4) elevated plus-maze (Andreatini R and L F Bacellar 1999 Braz J Med Biol Res 32(9): 1121-6), (5) defensive burying (Overmier J B et al., 1994 Biol Psychiatry 36(10): 703-4), and (6) the thirsty rat conflict (Mendelson W B et al., 1983 Life Sci 32(19): 2241-6; Overton D A et al., 1993 Psychopharmacology (Berl) 112(2-3): 270-6).

Vascular dementia can be evaluated using such models as vascular dementia rats (Cai, Chin J Integra Med. 2006 Dec;12(4):292-6) and the spontaneously hypertensive rats (SHR) (Sabbatini, Mach Ageing Dev. 2002 Mar 15;123(5):547-59). Each of the foregoing publications are incorporated herein by reference in their entirety.

Other evaluation methods routinely used in the art may be used to evaluate the effectiveness of the treatment methods described herein. Other evaluation methods include but are not limited to: Premorbid Intelligence Quotient Estimate, Mattis Dementia Rating Scale, Mini-Mental Status Exam, CVLT-II, CVLT, CVLT-II alternate, WMS-R, WMS-III, Hopkins Verbal Learning Test, Rey Complex Figure Test, Brief Visuospatial Memory Test, Continuous Visual Memory Test, Wisconsin Card Sorting Test, Stroop Color Word Interference Test, Trails B Army Intelligence Test Battery, Self-Ordered Pointing Test, WAIS-III Similarities Subtest, Boston Naming Test, Peabody Picture Vocabulary Test-III, Token Test, Digit Span, Hooper Visual Organization Test, Matrix Test Battery, Hamilton Depression Inventory, Beck Depression Inventory, and Modified Ranking Scale. One of ordinary skill in the art will appreciate which evaluation methods are appropriate for evaluating a particular neurological or mental disorder.

The invention will be explained in more detail below by way of examples, which illustrate the effectiveness of prototypical compound eltoprazine in alleviating specific symptoms associated with ADHD, which according to the method of the invention are indicative of efficacy for treating similar symptoms in other cognitive disorders. It is understood that the following examples and embodiments described herein are for illustrative purposes only and that various modifications or changes in light thereof will be suggestive to persons skilled in the art and are to be included within the spirit and purview of this application and the scope of the appended claims. Each publication, patent, and patent application cited herein is hereby incorporated by reference in its entirety for all purposes.

### EXAMPLE 1: The Effects of Eltoprazine on Symptoms of Attention Deficit Hyperactivity Disorder (ADHD) in Adults: A Double-Blind, Multiple-dose, Crossover, Safety and Preliminary Efficacy Trial

The primary objective of this study was to compare the effects of two doses of eltoprazine with placebo on symptoms of ADHD in adults. The primary efficacy parameter is the Attention-Deficit/Hyperactivity Disorder Rating Scale-IV (ADHD-RS-IV).

### Methodology:

This double-blind, multiple-dose, placebo-controlled, crossover study was conducted to assess the effects of 14 days of oral eltoprazine treatment in 48 adults with ADHD. Subjects eligible for study enrollment were to have met *Diagnostic and Statistical Manual of Mental Disorders, Fourth Edition, Text Revision* (DSM-IV-TR) criteria for ADHD. Assessment was to be made by clinical history, a Structured Clinical Interview for DSM-IV Axis Disorders-Research Version (SCID-RV), and a Conners' Adult ADHD Diagnostic Interview for DSM IV (CAADID).

Oral doses of eltoprazine hydrochloride were 5 mg/day or 10 mg/day for 14 consecutive days (i.e., 2.5 or 5 mg twice daily (bid), respectively). Eltoprazine hydrochloride was administered in gelatin capsules with calcium hydrogen phosphate, 2H₂O, maize starch NF, polyethylene glycol 6000 NF and magnesium stearate NF as excipients.

### Results:

### Primary Endpoint- ADHD-RS-IV

Primary endpoints were evaluated with the ADHD-RS-IV. The ADHD-RS-IV inattention, hyperactivity, and impulsivity subscale scores by treatment are shown in Table 1. After 14 days of treatment with placebo, 5 mg/day, or 10 mg/day eltoprazine, overall symptoms of ADHD were improved as measured by the ADHD-RS-IV, in which higher scores reflect greater impairment.

**Table 1: Results of ADHD-RS-IV Test; Inattention, hyperactivity, and impulsivity subscales**

| INATTENTION | Eltoprazine 5 mg/day N=38 | Eltoprazine 10 mg/day N=37 | Placebo N=38 |
|---|---|---|---|
| Baseline Score | 23.8 | 23.8 | 23.8 |
| Post Treatment Score | 14.5 | 14.8 | 17.0 |
| Change from Baseline | -9.4 (39%) | -9.0 (38%) | -6.8 (29%) |
| Eltoprazine vs. Placebo | P <0.041 | P <0.166 | |
| | | | |

| HYPERACTIVITY | Eltoprazine 5 mg/day N=38 | Eltoprazine 10 mg/day N=37 | Placebo N=38 |
|---|---|---|---|
| Baseline Score | 12.5 | 12.8 | 12.5 |
| Post Treatment Score | 6.6 | 7.2 | 8.1 |
| Change from Baseline | -5.9 (47%) | -5.5 (43%) | -4.4 (35%) |
| Eltoprazine vs. Placebo | P <0.047 | P <0.256 | |
| | | | |

| IMPULSIVITY | Eltoprazine 5 mg/day N=38 | Eltoprazine 10 mg/day N=37 | Placebo N=38 |
|---|---|---|---|
| Baseline Score | 6.1 | 6.1 | 6.1 |
| Post Treatment Score | 3.4 | 3.2 | 3.7 |
| Change from Baseline | -2.7 (44%) | -2.9 (47%) | -3.7 (39%) |
| Eltoprazine vs. Placebo | P <0.61 | P <0.44 | |

Total ADHD-RS-IV scores shown in Table 2 were decreased significantly with all treatments compared to baseline by 32%, 42%, and 41% with placebo, 5 mg/day, and 10 mg/day eltoprazine, respectively as shown in Table 2. Mean change from baseline for total ADHD-RS-IV scores were -13.6, -17.9, and -17.4 for placebo, 5 mg/day, and 10 mg/day, respectively (p<0.001 baseline vs. post-baseline for all treatments).

**Table 2: Results of ADHD-RS-IV Test, Total scores**

| | Eltoprazine 5 mg/day N=38 | Eltoprazine 10 mg/day N=37 | Placebo N=38 |
|---|---|---|---|
| Baseline Score | 42.4 | 42.7 | 42.4 |
| Post Treatment Score | 24.5 | 25.3 | 28.8 |
| Change from Baseline | -17.9 (42%) | -17.4 (41%) | -13.6 (32%) |
| Eltoprazine vs. Placebo | P <0.051 | P <0.180 | |

Using the ADHD-RS-IV inattention and hyperactivity subscales, statistically significant decreases were seen after 14 days of 5 mg/day eltoprazine compared to 14 days of placebo (p=0.041 and 0.047 for inattention and hyperactivity subscales, respectively). Inattention subscale scores were decreased by 29% with placebo and 39% with 5 mg/day eltoprazine, and hyperactivity subscale scores were decreased by 35% with placebo and 47% with 5 mg/day eltoprazine. Improvements were also seen for the impulsivity subscale although not to the same statistically significant level.

### Secondary Endpoint

Secondary endpoints were evaluated with the Clinical Global Impressions-Improvement (CGI-I) and Continuous Performance Test. (CPT). The CGI-I test is used to determine the patient's overall perception of improvement. Results of the CGI-I test are shown in Table 4. CGI-I scores were significantly improved with 10 mg/day eltoprazine. Seventy-one percent (71%) of subjects showed statistically significant improvement with 10 mg/day eltoprazine (p<0.029), compared to 62% with 5 mg/day eltoprazine and 52% with placebo (p<0.342 and 0.094, respectively) indicating that patient perception was that the treatment was effectively reducing symptoms. The CPT did not result in any statistically significant findings.

### Baseline ADHD-RS-IV Score >40

ADHD-RS-IV, CPT, and CGI-I scores were analyzed separately for those subjects whose baseline score was >40, indicating greater impairment. Twenty-two subjects had baseline ADHD-RS-IV total scores >40.

This subgroup analysis for ADHD-RS-IV scores is shown in Table 3 for total score, and inattention, hyperactivity, and impulsivity subscale scores. Mean ADHD-RS-IV total baseline scores were 47.2. By the end of 14 days of treatment, total ADHD-RS-IV scores were decreased significantly by 55% with 5 mg/day compared to a decrease of only 37% with placebo in subjects with more severe ADHD (p<0.029). The inattention, hyperactivity, and impulsivity subscale scores were also improved at 5 mg/day eltoprazine, but the change in score reached statistical significance compared to placebo only in the inattention subscale score (54% vs. 32% improvement 5 mg/day vs. placebo), and not in the hyperactivity and impulsivity subscale scores (56% and 55% (5 mg/day) vs. 39% and 42% (placebo) improvement, respectively).

**Table 3: Results of ADHD-RS Test, Total and Subscale Scores in Severe Patients**

| | ADHD-RS-Total | Inattention | Hyperactivity | Impulsivity |
|---|---|---|---|---|
| All patients (n=36) (5 mg) | 0.051 | 0.041 | 0.047 | 0.61 |
| All patients (n=36) (10 mg) | 0.180 | 0.166 | 0.256 | 0.44 |
| Severe Patients (n=22) (5 mg) | 0.029 | 0.016 | 0.059 | 0.31 |
| Severe Patients (n=22) (10 mg) | 0.805 | 0.792 | 0.884 | 0.80 |

This subgroup analysis for CGI-I scores is shown in Table 4. By the end of 14 days of treatment, 62% and 71% of subjects showed improvement with 5 and 10 mg/day eltoprazine, respectively, compared to 52% with placebo. The difference between treatment groups was similar (p<0.190 and 0.091 for eltoprazine vs. placebo for 5 and 10 mg/day, respectively). While the improvement with 10 mg/day eltoprazine was statistically significant (p=0.029), the improvement seen with placebo and 5 mg/day eltoprazine did not reach statistically significant levels (p<0.094 and 0.342, respectively). The CPT did not result in any statistically significant findings.

**Table 4: Results of CGI-I test**

| | Eltoprazine 5mg n=38 | Eltoprazine 10mg n=37 | Placebo n=38 |
|---|---|---|---|
| Mean Improvement (S.D.) | 2.6(1.2) | 2.7(0.9) | 3.2(1.2) |
| Eltoprazine vs. Placebo* | P<0.19 | P<0.09 | |
| | | | |
| Number of Patients Improved | 26 | 30 | 22 |
| Number of Patients Not Improved | 12 | 7 | 16 |
| P value** | 0.34 | 0.03 | |

### Conclusions

The most interesting finding of this study was the selective action of eltoprazine predominantly on inattention and hyperactivity and to a lesser degree impulsivity. The primary efficacy endpoint was change from baseline in ADHD-RS-IV score between 5 or 10 mg/day eltoprazine and placebo. After 14 days of treatment with placebo, 5 mg/day, or 10 mg/day eltoprazine, overall symptoms of ADHD were improved compared to baseline as measured by the ADHD-RS-IV. Total ADHD-RS-IV scores declined significantly (i.e., lower scores equal less impairment) by 32%, 42%, and 41% from baseline with placebo, 5 mg/day, and 10 mg/day eltoprazine, respectively. However, these changes in total ADHD-RS-IV were not significant between placebo and eltoprazine (5 or 10 mg/day). When assessed using the ADHD-RS-IV inattention and hyperactivity subscales, there was a statistically significantly greater decline from baseline score (i.e., greater improvement in symptoms) with 5 mg/day eltoprazine compared to placebo treatment. Impulsivity score also improved, but was not to a statistically significant degree.

Secondary endpoints were changed from baseline for CGI-I scores between 5 or 10 mg/day eltoprazine and placebo. CGI-I scores were significantly improved from baseline with 10 mg/day eltoprazine, but these scores were not statistically significantly different between placebo and eltoprazine treatment.

ADHD-RS-IV, and CGI scores were analyzed separately for those subjects whose baseline score was > 40, indicating greater impairment. In this subpopulation with more severe ADHD, greater improvement was seen in ADHD symptoms with 5 mg/day eltoprazine. Total ADHD-RS-IV scores decreased from baseline by 55% and subscale scores decreased by 54%, 56, and 55% for inattention, hyperactivity, and impulsivity, respectively. CGI-I scores showed improvement in ADHD symptoms in the more severely affected population with 5 mg/day eltoprazine (14% reduction in severity score and 62% of subjects with improvement, respectively). These changes with 5 mg/day eltoprazine were statistically significantly different from placebo for the ADHD-RS-IV total and inattention subscore. While the 10 mg/day eltoprazine dose also showed improvement in symptoms, the results were statistically significantly different from placebo for the CGI-I.

Examples 2 through 12 are prophetic to illustrate how one of ordinary skill in the art would carry out the methods described herein to treat symptoms or conditions that are associated with cognitive impairment. These examples do not limit the scope of the invention and similar methods may be applied to other symptoms and conditions related to cognitive impairment or symptoms such as inattention, hyperactivity or impulsivity.

### EXAMPLE 2: Treatment of Inattention and Hyperactivity in Schizophrenia with Eltoprazine

Patients presenting clinically with symptoms of schizophrenia such as delusions, hallucinations, and disorganized or altered speech according to the DSM-IV are evaluated with conventional testing using Eye Tracking Dysfunction and Impaired Prepulse Inhibition and the MATRICS test battery as well as the CGI-I and ADHD-RS-IV inattention and hyperactivity subscales and other tests routinely used by mental health professionals to evaluate schizophrenia.

Patients are prescribed 10 mg/day eltoprazine and then evaluated again after 2 weeks to determine if symptoms have improved. After evaluation, the dosage is adjusted up or down or kept the same depending on the change in the symptoms of schizophrenia. The treatment is maintained for as long as necessary to effect a stable resolution of the symptoms of schizophrenia.

### EXAMPLE 3: Treatment of Depression with Eltoprazine

Patients presenting clinically with symptoms of depression according to the DSM-IV are evaluated with conventional testing using behavioral tests such as Hamilton Depression Inventory, Beck Depression inventory.

Patients are prescribed 5mg/day eltoprazine and then evaluated again after 2 weeks using the same behavioral tests whereupon dosage is adjusted up or down or kept the same depending on the individual response to the initial dosage. Administration is maintained for as long as necessary to ameliorate symptoms of depression.

### EXAMPLE 4: Treatment of Psychomotor Retardation with Eltoprazine

Patients that are diagnosed with psychomotor retardation characterized by slowing of coordination, speech, and impaired articulation are evaluated using the Psychomotor Vigilance Test (PVT) and computerized digit symbol substitution test (CDSST) (Rogers et al. Brain, Vol. 110, No. 3, 761-776, 1987).

Patients are prescribed 5mg/day eltoprazine (bid) orally and vital signs including orthostatic heart rate and blood pressure are measured 2-4 hours after dose to assess tolerance of the compound. Supine heart rate and blood pressure are to be determined after a 5-minute rest and standing heart rate and blood pressure after 1-2 minutes erect. Subjects are to have an electrocardiogram (ECG) recorded 2-4 hours after drug administration. Patients are then evaluated again after 2 weeks using the PVT and CDSST. At each evaluation, the dosage may be adjusted up or down or kept the same depending on the individual response to the initial dosage. Administration is maintained until psychomotor retardation is reduced.

### EXAMPLE 5: Treatment of Inattention and Hyperactivity with Eltoprazine In Parkinson's Disease

Patients that are diagnosed clinically with Parkinson's disease are evaluated using the CGI-I and ADHD-RS-IV inattention and hyperactivity subscales.

Patients are prescribed 5 mg/day eltoprazine bid and then evaluated again after 2 weeks using the same behavioral tests whereupon dosage is adjusted up or down or kept the same depending on the individual response to the initial dosage. Administration is maintained for as long as necessary to treat inattention.

### EXAMPLE 6: Improvement of Functional Recovery After Stroke

Stroke patients are assessed on admission and at 2, 4, and 6 months after stroke with the Barthel Index, Rivermead Motor Assessment of Gross Function, Rivermead Motor Assessment of Leg/Trunk, Rivermead Motor Assessment of Arm, and Nottingham Extended Activities of Daily Living (except on admission).

Patients are prescribed 5mg/day eltoprazine (bid) orally and vital signs including orthostatic heart rate and blood pressure are measured 2-4 hours after dose to assess tolerance of the compound. Supine heart rate and blood pressure are to be determined after a 5-minute rest and standing heart rate and blood pressure after 1-2 minutes erect. Subjects are to have an electrocardiogram (ECG) recorded 2-4 hours after study drug at days 7 and 35, as well at screening and day 45/end-of-study. Patients are then evaluated again after 2 weeks, 1 month and 3 months using the same tests. Eltoprazine will improve cognitive function during the rehabilitative treatment when the patient is undergoing training and relearning of common tasks.

### EXAMPLE 7: Treatment of Alzheimer's Disease with Eltoprazine

Consecutive outpatients diagnosed with Alzheimer's disease, in accordance with Diagnostic and Statistical Manual of Mental Disorders (4^{th} Ed.) are evaluated using the Mini-Mental State Examination (MMSE) and other tests to assess memory, reasoning, vision-motor coordination and language skills. Particular attention is paid to cognitive functions dealing with attention, working memory, judgment, and decision-making.

Patients are prescribed 5mg/day eltoprazine and then evaluated again after 2 weeks using the same behavioral tests whereupon dosage is adjusted up or down or kept the same depending on the individual response to the initial dosage. Administration is maintained for as long as necessary to ameliorate symptoms of Alzheimer's disease and improve attention, judgment, and decision-making.

### EXAMPLE 8: Treatment of Anxiety Disorders with Eltoprazine in Juvenile Patients

Juvenile patients meeting DSM-III-R or DSM-IV criteria for an anxiety disorder are evaluated with the Clinical Global Impression of Improvement (CGI-I) and the Hamilton Anxiety Rating Scale (HARS).

Patients are prescribed 5mg/day eltoprazine and then evaluated again after 2 weeks using the same behavioral tests whereupon dosage is adjusted up or down or kept the same depending on the individual response to the initial dosage. The children are judged "much" or "very much improved" at post intervention on number of anxiety diagnoses, number of DSM-IV anxiety symptoms, and ability to cope with feared situations. Administration is maintained for as long as necessary to ameliorate symptoms of anxiety disorder.

### EXAMPLE 9: Treatment of Cognitive Disinhibition with Eltoprazine

Patients that are diagnosed clinically with cognitive disinhibition, characterized by sensation seeking and impulsive decision-making, are evaluated using the Hayling Sentence Completion Test. The Disinhibition-scale of Zuckerman's Sensation Seeking Scale is used to differentiate between low vs. high in cognitive disinhibition and the Matching Familiar Figures Test (Kagan et al., 1964) is used to obtain an index of behavioral inhibition.

Patients are prescribed 5 mg/day eltoprazine bid and then evaluated again after 2 weeks using the same behavioral tests whereupon dosage is adjusted up or down or kept the same depending on the individual response to the initial dosage. Administration is maintained for as long as necessary to treat cognitive disinhibition.

### EXAMPLE 10: Treatment of Schizophrenia-Associated Hyperactivity with Eltoprazine

Patients presenting clinically with symptoms of schizophrenia such as delusions, hallucinations, and disorganized or altered speech according to the DSM-IV, are evaluated using the CGI-I and ADHD-RS-IV inattention and hyperactivity subscales.

Patients are prescribed 5 mg/day eltoprazine bid and then evaluated again after 2 weeks using the same behavioral tests whereupon dosage is adjusted up or down or kept the same depending on the individual response to the initial dosage. Administration is maintained for as long as necessary to treat hyperactivity.

### EXAMPLE 11: Treatment of Inattention with Eltoprazine in Patients with Alzheimer's Disease

Patients presenting clinically with symptoms of Alzheimer's Disease, are evaluated using the CGI-I, CPT and ADHD-RS-IV inattention and hyperactivity subscales.

Patients are prescribed 5 mg/day eltoprazine bid and then evaluated again after 2 weeks using the same behavioral tests whereupon dosage is adjusted up or down or kept the same depending on the individual response to the initial dosage. Administration is maintained for as long as necessary to treat inattention.

### EXAMPLE 12: Treatment of Inattention, Hyperactivity and Impulsiveness with Eltoprazine in Schizophrenia Patients Without Co-morbid ADHD

Patients presenting clinically with symptoms of schizophrenia such as delusions, hallucinations, and disorganized or altered speech according to the DSM-IV, are evaluated using the CGI-I, CPT and ADHD-RS-IV inattention, hyperactivity and impulsivity subscales.

Patients are prescribed 5 mg/day eltoprazine bid and then evaluated again after 2 weeks using the same behavioral tests whereupon dosage is adjusted up or down or kept the same depending on the individual response to the initial dosage. Administration is maintained for as long as necessary to treat inattention, hyperactivity and impulsivity.

### EXAMPLE 13: The effect of eltoprazine on attention and impulsivity

The Timing/Peak Procedure test is an operant test in which mice are trained to respond to a food reward at a fixed time interval of 30 sec. Mice learn to increase their responding around the 30 sec time period. This test assesses impulsivity, attention and timing perception. A high peak of responding at the 30 sec interval and narrow spread are signatures of the animal's improved attention and time perception.

In brief, mice were placed in a chamber and trained to lever press for food. After training, animals learn to respond after a fixed interval of 30 sec has elapsed as only this fixed interval response produces a reward. With reinforcement delivery the lever is retracted and an inter-trial-interval commences. Once the animal was trained on the reinforced trials, "peak trials" or unreinforced trials are introduced and intermixed with reinforced trials. During these empty trials, no responses were reinforced and the trial lasts for 120 sec (i.e., the lever is extended but reinforcement is not presented). After the 120 sec elapses, the trial terminated, the lever was retracted and the inter-trial-interval started as before. The animals therefore learned that if after a period of 30 sec, no food has been presented there will be no reinforcement delivery until the lever is retracted and the trial starts again.

To perform well on this task, animals need to learn an association between a response (lever pressing) and the delivery of reinforcement (condensed milk), they need to perceive and remember time, they need to act on the remembered time by responding or by inhibiting a response and finally they need to compare the elapsed time during a trial with their memory for the time for reinforcement. A true cognitive enhancer therefore should decrease responding after reinforcement time, without affecting the peak time, i.e., sharpening the curve and reducing the spread in the response curve. A sharpened curve is indicative of heightened attentional processes.

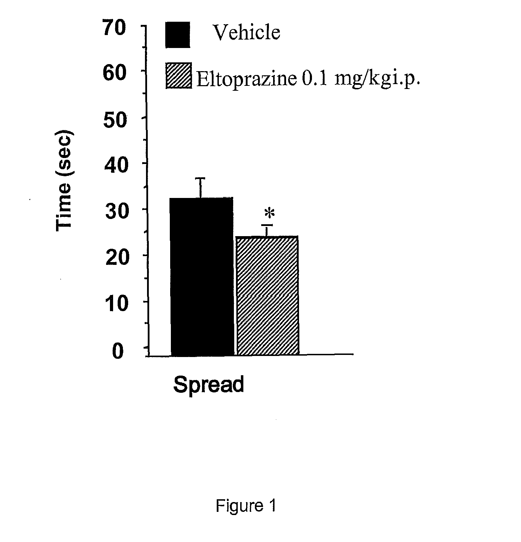

See FIG. 1 for data demonstrating that eltoprazine (0.1mg/kg IP) sharpened the peak of responding and significantly reduces the spread. There was no loss of effect following chronic administrations.

### EXAMPLE 14: The Effect of Eltoprazine on Working Memory

Several mazes, such as the radial arm maze (RAM) have been adapted to assess working memory in rodents. The RAM consists of eight long runways radiating out in a circle from a central compartment. In the RAM rats learn to retrieve food rewards from all or a sub-set of the arms. Working memory is assessed by measuring entries into previously baited arms within a given trial. The RAM has been found to provide a sensitive assessment of working memory in the rat. Hippocampal lesions produce deficits both in adults and in the neonatal hippocampal lesion model of schizophrenia.

For training, food deprived rats were placed in an enclosed cylinder in the center of the maze for 20 s, prior to being allowed to explore the maze by removing the cylinder. Each radial arm is baited with a food reward. Animals were given a maximum of 5 minutes to retrieve all food rewards. This training continued once daily for three weeks until animals met the performance criteria for successful acquisition of the task.

Four parameters were recorded to assess the performance of the rat: (1) the number of errors, which is defined as entering an arm that had already been entered, (2) the number of consecutive entries into un-visited arms before the first error occurred (entries to repeat), (3) latency to complete the task, and (4) the total number of arm entries before all food in the eight arms is collected or the maximum session duration is reached. The criteria for successful acquisition of the task are a maximum of three errors and a minimum of 6 entries to repeat, per session for three consecutive session. For drug tests, all eight arms of the radial arm maze were baited. The session continued until all the food pellets were collected or 10 minutes has passed, whichever occurred first.

Rats received scopolamine (0.5 mg/kg IP) + vehicle or scopolamine (0.5 mg/kg IP) + eltoprazine (0.3 mg/kg IP), according to a cross-over design. All subjects received both drug treatments. Scoring of behavioral performance and drug administration were performed by an experimenter blinded to the treatment conditions. All drug test sessions were captured on video-tape.

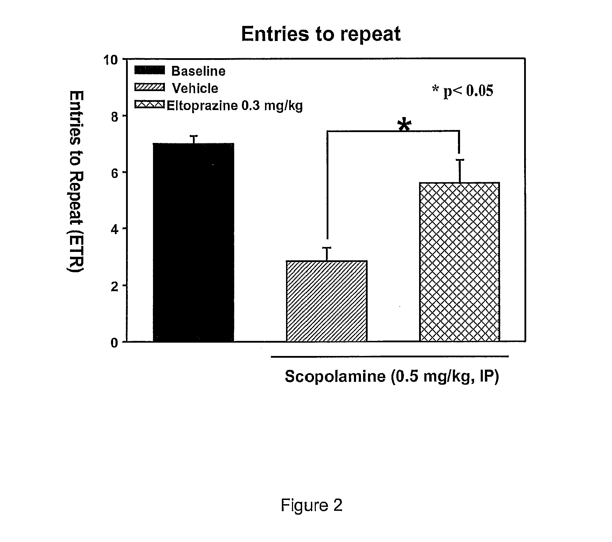

Administration of 0.5 mg/kg IP scopolamine resulted in decreased entries to repeat in the radial arm maze compared to baseline, and this effect was attenuated by administration of 0.3 mg/kg PO eltoprazine, as revealed by ANOVA. See FIG. 2.

### EXAMPLE 15: The Effect of Eltoprazine on Visual-Spatial Memory

In addition to working memory deficits, the initiative, "Measurement and Treatment Research to Improve Cognition in Schizophrenia" (MATRICS) has identified other domains of learning and memory in which schizophrenia patients show impaired visual learning and memory. There are several visual learning and memory tasks appropriate for study in rodents (Powell, S.B. and M.A. Geyer, Overview of animal models of schizophrenia. Curr Protoc Neurosci, 2007. Chapter 9: p. Unit 9 24). One memory test in rodents that has a visual-spatial component is the Novel Object Recognition Test (NOR) (Ennaceur, A. and J. Delacour, A new one-trial test for neurobiological studies of memory in rats. 1: Behavioral data. Behav Brain Res, 1988. 31(1): p. 47-59). The test involves exposing rats to two identical objects and, following a delay, placing the rats back in the chamber with one of the familiar objects it encountered in the first exposure and an additional novel object. A rodent's natural tendency will be to explore the novel object more than the familiar object. Therefore, rats that spend a longer time exploring the novel object are considered to have a better "memory" of the familiar object encountered in the original exposure.

Male Wistar rats were assessed for cognitive ability in a test apparatus comprising an open-field arena placed in a sound-attenuated room under dimmed lighting. Each rat was tested separately and care was taken to remove any olfactory/taste cues by cleaning the arena and test objects between trials and rats. All tests were video scored by an observer blind to treatment. On Day 1, rats were allowed to freely explore the arena for a 10-minute habituation period. On Day 2, each rat was placed into the test arena in the presence of two identical objects. Each rat was placed in the arena facing the same direction at the same position, and the time spent actively exploring the objects during a 10-minute test period was recorded. The rat was returned to its home cage between tests. After 24 hours, each rat was placed again in the test arena for 10 minutes in the presence of one of the familiar objects and a novel object, and the time spent exploring both objects was recorded. The presentation order and position of the objects (left/right) was randomized between rats to prevent bias from order or place preference. The percent time spent exploring the novel object was recorded.

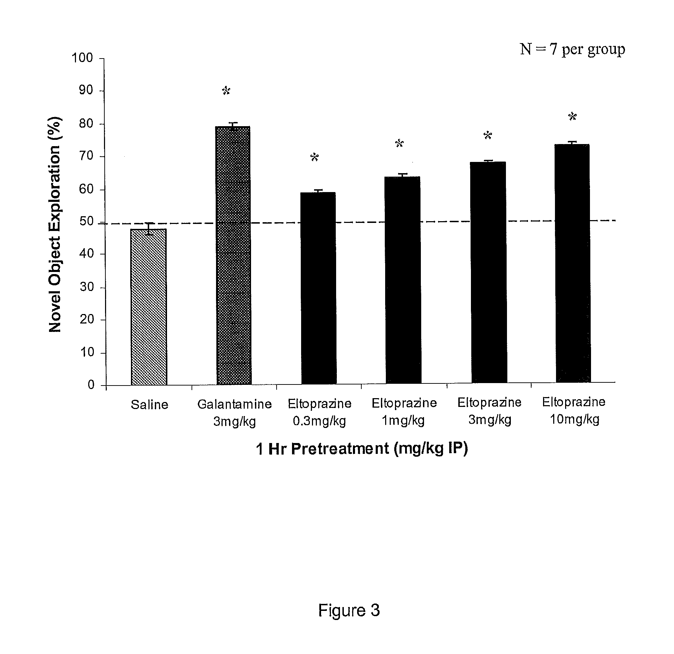

Eltoprazine at 0.3, 1, 3, and 10 mg/kg administered 1 h prior to training significantly improved rat novel object recognition (NOR) memory as compared to vehicle-treated controls 24 hours post training. The positive control galantamine, at 3 mg/kg, also significantly increased NOR memory relative to saline. See FIG. 3.

### EXAMPLE 16: Microdialysis Experiments

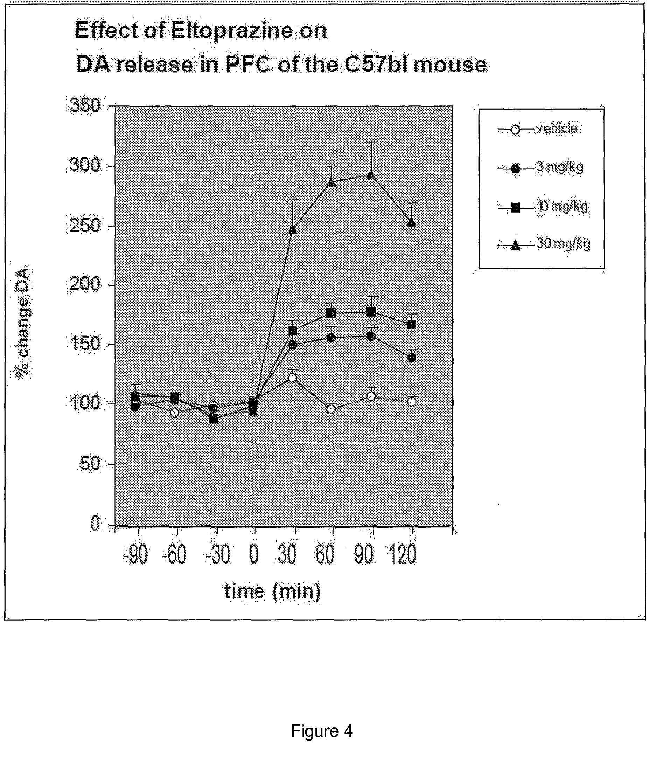

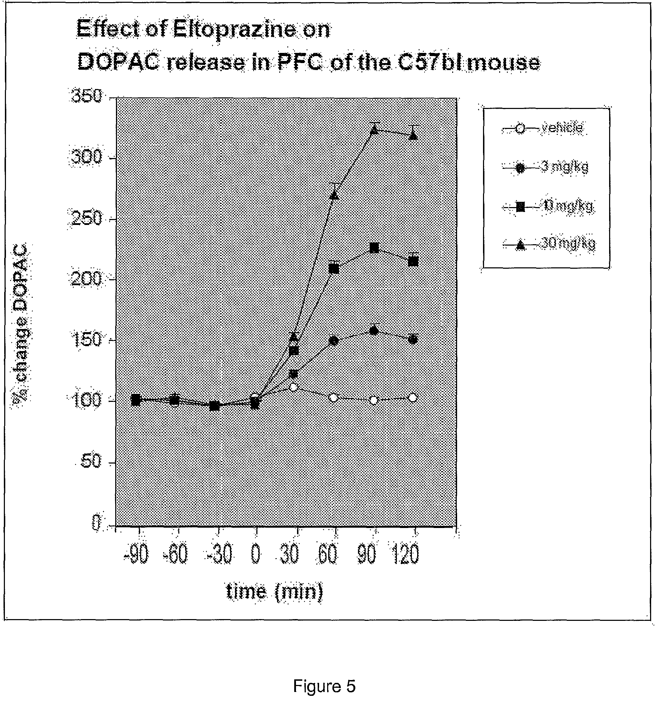

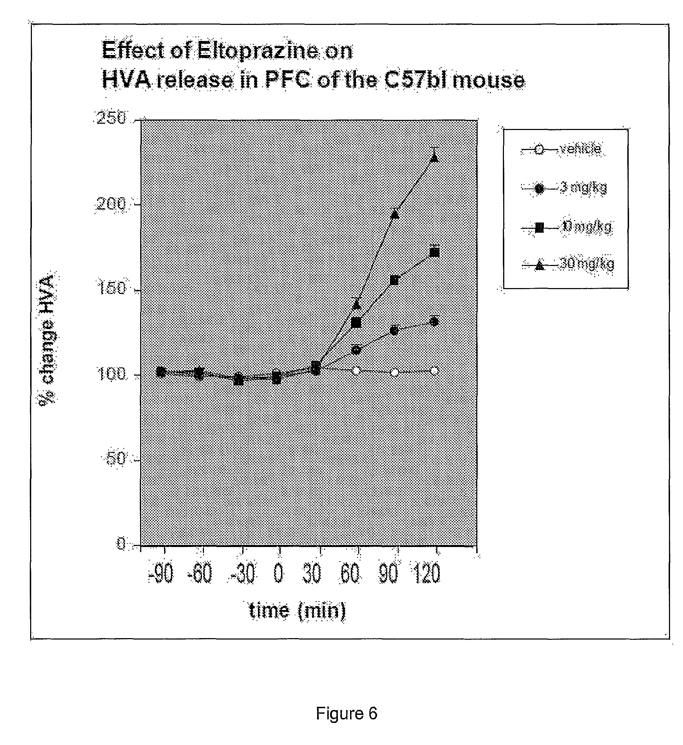

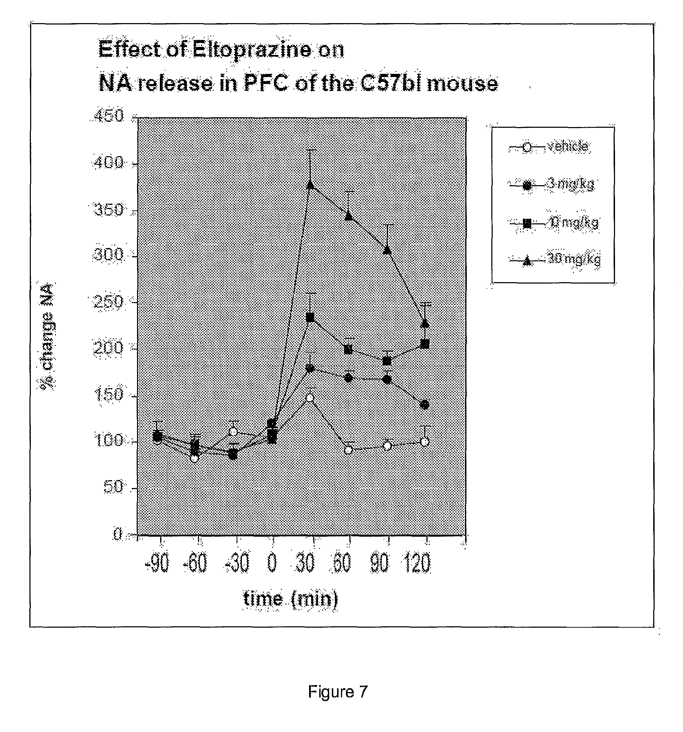

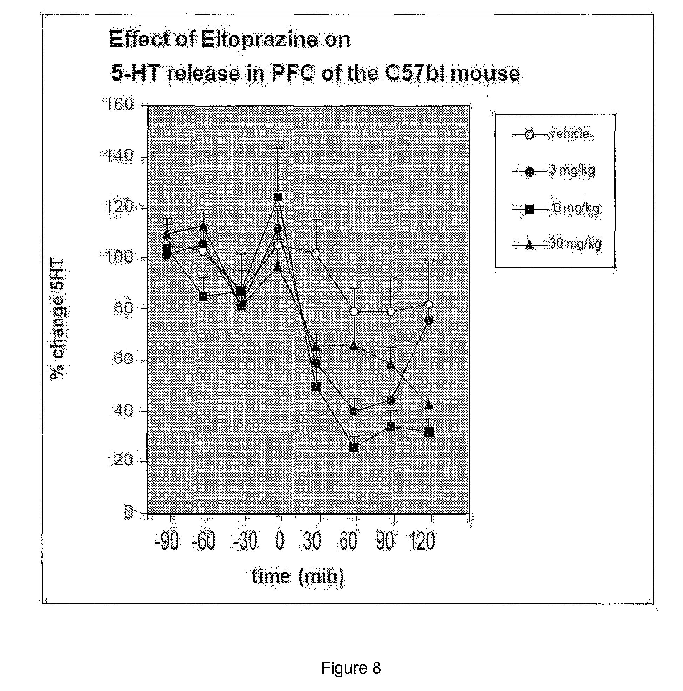

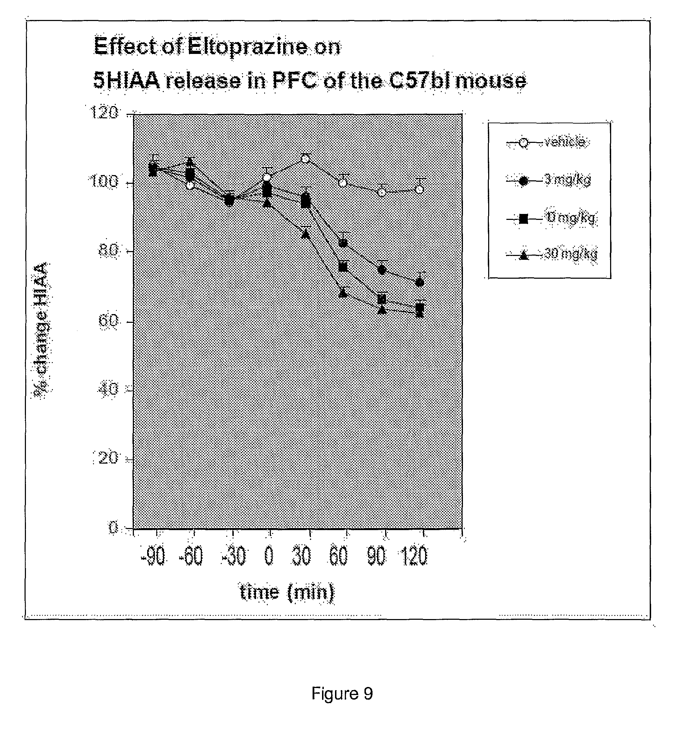

There is accumulating evidence that elevated dopamine function in the prefrontal cortex (PFC) may improve cognitive deficits in schizophrenic patients. Eltoprazine produces a significant release of dopamine and norepinepherine in the PFC and a corresponding decrease in the release of serotonin (see FIGS. 4 - 9).

Microdialysis probes were implanted in the prefrontal cortex; coordinates were: AP +2.0 mm, ML +0.7 mm (under an 8° angle) from bregma, DV -3.3 mm from the dura, with the tooth-bar also set at 0 mm. The active dialysis surface length of the membrane was 2 mm. Microdialysis experiments started 48 h (prefrontal cortex) after surgery. Ringer solution was perfused through the microdialysis probe at a flow at 1.166 µl/min using a high precision pump (KdScientific 220). Mouse dual channel swivels (Type 375/D/22QM, Instech Laboratories, Inc.) connected to PEEK-tubing (ID 0.005," OD 0.020") were used to allow unrestrained movements of the mice. Samples were collected by hand 2.5 h after the start of the dialysis probe perfusion to obtain stable baseline values for the monoamines and their metabolites. The average of the first four samples was calculated and represents the baseline level. Samples were collected every 30 min were analyzed by HPLC with electrochemical detection.

Six mice per dose group were treated with vehicle or eltoprazine at 3 mg/kg, 10 mg/kg and 30 mg/kg. Eltoprazine exhibited a dose-related increase in the release of dopamine and norepinephrine and their metabolites (DOPAC and HVA) and corresponding decrease in serotonin and its metabolite (5HIAA) in the PFC of C57 mice. See FIGS. 4 - 9.

## Claims

1. Eltoprazine or a pharmaceutically acceptable salt thereof for use in treating Parkinson's disease, said treatment comprising administering to a subject with Parkinson's disease from 0.1 mg/day to 10 mg/day of eltoprazine or a pharmaceutically acceptable salt thereof, wherein said subject has a movement disorder, said subject also being treated with an additional therapeutic drug to alleviate symptoms of Parkinson's disease.

2. Eltoprazine or a pharmaceutically acceptable salt thereof for use according to claim 1, wherein from 0.5 mg/day to 5 mg/day of said eltoprazine or a pharmaceutically acceptable salt thereof is administered to said subject.

3. Eltoprazine or a pharmaceutically acceptable salt thereof for use according to claim 1, wherein from 2.5 mg/day to 5 mg/day of said eltoprazine or a pharmaceutically acceptable salt thereof is administered to said subject.

4. Eltoprazine or a pharmaceutically acceptable salt thereof for use according to claim 1, wherein 2.5 mg/day of said eltoprazine or a pharmaceutically acceptable salt thereof is administered to said subject.

5. Eltoprazine or a pharmaceutically acceptable salt thereof for use according to any one of claims 1-4, wherein said additional therapeutic drug is Levodopa.

6. Eltoprazine or a pharmaceutically acceptable salt thereof for use according to any one of claims 1 to 5, wherein said eltoprazine or a pharmaceutically acceptable salt thereof is administered once per day.

7. Eltoprazine or a pharmaceutically acceptable salt thereof for use according to any one of claims 1 to 5, wherein said eltoprazine or a pharmaceutically acceptable salt thereof is administered twice per day.

8. Eltoprazine or a pharmaceutically acceptable salt thereof foruse according to claim 1, wherein more than 50% of said eltoprazine or pharmaceutically acceptable salt thereof is administered at the beginning half of the day.
